# EUROPÄISCHE PATENTSCHRIFT

(11) **EP 2 324 755 B1**
(45) Veröffentlichungstag und Bekanntmachung des Hinweises auf die Patenterteilung: **25.07.2012**
(21) Anmeldenummer: 10191474.5
(22) Anmeldetag: 17.11.2010
(51) Int. Cl.: A61B 1/005

(54) **Endoskop mit flexiblem Endoskopschaft**
Endoscope with flexible endoscopic shaft
Endoscope doté d'un arbre d'endoscope flexible

(30) Priorität: 19.11.2009 DE 102009054367; 30.04.2010 DE 202010006478 U
(43) Veröffentlichungstag der Anmeldung: 25.05.2011
(73) Patentinhaber: Karl Storz GmbH & Co. KG, 78532 Tuttlingen (DE)
(72) Erfinder: Schwarz, Peter, 78532, Tuttlingen-Nendingen (DE)
(74) Vertreter: Witte, Weller & Partner

(56) Entgegenhaltungen:
- US-A- 5 159 446
- US-A- 5 989 182
- US-A1- 2006 069 310

## Beschreibung

Die Erfindung betrifft ein Endoskop, das einen flexiblen Endoskopschaft mit einem ablenkbaren distalen Endabschnitt aufweist, mit einem mechanischen Aktuator, der in einem proximalen Endbereich des Endoskopschafts angeordnet ist und der verschiedene Geschwindigkeitsstufen besitzt, mit zumindest einem Zugelement, das mit dem distalen Endabschnitt und mit dem Aktuator mechanisch gekuppelt ist, wobei der Aktuator das Zugelement für eine Ablenkbewegung des distalen Endabschnitts in eine Zugrichtung bewegt, und mit einer Steuereinheit, die dazu ausgebildet ist, den Aktuator in seinen Geschwindigkeitsstufen zu steuern.

Ein derartiges Endoskop ist aus der US-A-5 400 769 bekannt.

Endoskope mit flexiblen Endoskopschäften werden sowohl in der Industrie als auch im medizinischen Bereich verwendet. Beispielsweise werden flexible Endoskope im veterinär-medizinischen Bereich bei der gastroendoskopischen Untersuchung von Großtieren eingesetzt. Diese Endoskope besitzen an einem distalen Ende ihres Endoskopschafts einen distalen Endabschnitt, der in einem Endstück endet. Das Endstück stellt den Teil des Endoskops dar, welcher in den zu untersuchenden Körper eingeführt wird. Es weist typischerweise das distale Ende einer Endoskopoptik sowie Absaug-, Spül- und Werkzeugkanäle auf. Um eine größtmögliche Flexibilität bezüglich einer räumlichen Ausrichtung des Endstücks bei der Untersuchung gewährleisten zu können, ist der Endabschnitt des Endoskopschafts typischerweise ablenkbar ausgebildet. Durch Ablenken, z.B. Abkrümmen, des Endabschnitts relativ zu dem restlichen Endoskopschaft, kann das Endstück nach Wunsch ausgerichtet werden. Bei diesem Ablenken ist eine vorsichtige Vorgehensweise eines Anwenders notwendig, damit kein Gewebe verletzt wird, das sich um den Endabschnitt herum befindet. Somit ist es wichtig, dass das Ablenken des Endabschnitts sehr präzise steuerbar ist.

Die eingangs genannte US-A-5 400 769 beschreibt ein Endoskop mit einem flexiblen Endoskopschaft. Der Endoskopschaft weist einen ablenkbaren distalen Endabschnitt auf, der dazu dient, ein Endstück auszurichten. Um das Endstück auszurichten, ist ein Aktuator in Form eines Elektromotors vorgesehen, der mittels einer Kette als Zugelement den Endabschnitt ablenken kann. Weiter ist eine Steuereinrichtung vorgesehen, die dafür sorgt, dass das Ablenken des Endabschnitts mit konstanter Geschwindigkeit durchgeführt wird. Das bedeutet, dass das Endstück hier während des Ablenkens mit konstanter Geschwindigkeit bewegt wird. Um das zu erreichen, ist vorgesehen, dass eine elektrische Spannung, die den Elektromotor versorgt, in Abhängigkeit eines Ablenkwinkels des Endabschnitts geregelt wird. Der Ablenkwinkel bildet sich zwischen einer aktuellen Ausrichtung des Endstücks und einer Geradeausstellung des Endstücks aus. Die Geradeausstellung ist dabei die Ausrichtung des Endstücks, in der der Endabschnitt gerade ausgerichtet ist. Bei großen Ablenkwinkeln ist eine große Kraft zum weiteren Ablenken des Endabschnitts nötig, weshalb eine größere elektrische Spannung eingesetzt wird als bei kleineren Ablenkwinkeln, um die Ablenkgeschwindigkeit konstant beizubehalten. Es wird weiter vorgeschlagen, die Geschwindigkeit des Elektromotors dann zu erhöhen, wenn der Ablenkwinkel sehr klein ist, also der Endabschnitt im Wesentlichen gerade ausgerichtet ist. Dies wird damit begründet, dass insbesondere in diesem Bereich um die Geradeausstellung eine konstruktionsbedingte Spannungslosigkeit des Zugelements vorliegt. Eine mögliche Anlauftotzeit oder ein möglicher Nachlaufweg beim Ablenken des Endabschnitts bleibt von diesem Stand der Technik unberührt.

Die US 2006-069310 beschreibt eine "fly by wire" Steuerung eines Endoskops und erwähnt einen "rapid deceleration" Schritt bei Erreichen der Zielauslenkung.

Das Ablenken des Endabschnitts wird typischerweise durch die Verwendung von Bowdenzügen realisiert. Der flexible Endoskopschaft bildet zusammen mit den Bowdenzügen ein Feder-Dämpfer-System. Dabei stellt der Bowdenzug ein Federelement dar, und die Reibung an den Bowdenzügen sowie die Ablenkung des distalen Endabschnitts stellt den Dämpfer dar. Beim Aufbringen einer Zugkraft in eine proximale Richtung an einem proximalen Ende des Bowdenzuges wird dieser relativ zu dem Endoskopschaft verlagert. Der erste Teil des Verlagerns wird vollständig für das Spannen des Feder-Dämpfer-Systems benötigt. Somit ergibt sich eine Anlauftotzeit, bei der der Bowdenzug verlagert wird, aber noch kein Ablenken des Endabschnitts erfolgt. Erst wenn die Federkraft so groß ist, dass die Reibungen überwunden werden können, beginnt sich der Endabschnitt zu bewegen. Ab diesem Punkt erfolgt die Ablenkbewegung in Abhängigkeit von der Bewegung des Bowdenzugs. Wird die Bewegung des Bowdenzugs anschließend gestoppt, wird also der Bowdenzug in seiner Position festgehalten, dann entlädt sich die im Federsystem gespeicherte Energie durch Entspannen. Dabei neigt der Endoskopschaft dazu, sich gerade auszurichten. Das führt zu einem weiteren Spannen des Bowdenzugs. Insgesamt ergibt sich, dass der Bowdenzug relativ zu dem Endoskopschaft verkürzt wird. Der dabei benötigte Weg des Bowdenzugs kann in dem gesamten System nur durch weiteres Ablenken des Endabschnitts gewonnen werden, wodurch ein Nachlaufweg des Endabschnitts und damit des Endstücks erfolgt.

Für den Anwender des Endoskops ergibt sich somit, dass nach Ende des Ablenkens das Endstück aufgrund des Nachlaufweges nicht so ausgerichtet ist, wie es der Anwender wünscht. Der Nachlaufweg führt also dazu, dass der Anwender nicht die Stelle durch das Endoskop sieht, die er eigentlich sehen möchte. Zudem erkennt er zu Beginn des Ablenkens aufgrund des Anlauftotweges keine Wirkung an dem Endstück, was die Handhabung zusätzlich erschwert.

Bei einer manuellen Ablenkung eines Endoskops, wie dies beispielsweise mittels eines Handrads möglich ist, kann das Handrad zunächst nahezu widerstandslos um einen bestimmten Winkel gedreht werden, bevor sich der Endabschnitt tatsächlich ablenken lässt. Nachdem die Drehung am Handrad gestoppt ist, erfolgt ein Nachlauf des Endabschnitts. Die Anlauftotzeit und der Nachlaufweg führen so zu einer ungenauen Steuerung des Endabschnitts und damit der Ausrichtung des Endstücks, da der Anwender ständig die Anlauftotzeit und den Nachlaufweg bei einer Bedienung des Handrads berücksichtigen muss. Bei manueller Betätigung hat der Anwender jedoch den Vorteil, dass er die Gegenkraft des Handrads spürt und dadurch die Anlauftotzeit sowie den Nachlaufweg minimieren kann.

JP 06-022904 A beschreibt ein Endoskop, das einen flexiblen Endoskopschaft sowie einen ablenkbaren distalen Endabschnitt aufweist. Der Endabschnitt kann mittels zweier Motoren über Zugdrähte abgelenkt werden. Es soll ein Durchhängen der Zugdrähte verhindert werden, so dass das Ablenken des distalen Endabschnitts ohne Verzögerung erfolgt. Hierzu wird vorgeschlagen, den entsprechenden Motor so lange in einer hohen Geschwindigkeit zu betreiben, bis der Zugdraht gespannt ist. Anschließend wird die Ablenkung des Endabschnitts in einer normalen Geschwindigkeit vorgenommen. Ein Entspannen der Zugdrähte zum Ende einer Ablenkbewegung wird davon nicht berührt.

JP 2000-300511 A beschreibt ein Endoskop mit einem flexiblen Schaft in einem ablenkbaren distalen Endabschnitt. Es weist eine Steuereinrichtung auf, die einen Motor dann in einer anderen Geschwindigkeit betreibt, wenn eine fehlende Spannung eines Zugelements ausgeglichen werden muss. Zudem wird vorgeschlagen, dass der Motor dann schneller bewegt wird, wenn der ungespannte Zustand vorliegt, um diesen möglichst schnell zu überwinden. Im Anschluss an die schnellere Bewegung wird der Motor wieder in einer normalen Geschwindigkeit betrieben.

US 7,348,751 B2 beschreibt ein Endoskop, das einen ablenkbaren distalen Endabschnitt aufweist. Es ist eine Lageregelung des distalen Endabschnitts beschrieben, wobei unterschiedliche Regelkreise auf Basis verschiedener Messeinrichtungen vorgeschlagen werden. Insbesondere ist vorgesehen, dass eine Spannungslosigkeit von Zugdrähten im Bereich einer Geradeausstellung des distalen Endabschnitts vermieden wird, so dass der Endabschnitt ohne Verzögerung ausgelenkt werden kann. Um dies zu erreichen, wird vorgeschlagen, eine Korrekturtabelle einzusetzen, welche bewirkt, dass der Motor dann beschleunigt wird, wenn der Zugdraht ungespannt ist.

Die US 6,669,629 beschreibt eine automatische Positionsregelung eines Endstücks sowie eine Reduktion von mechanischen Spannungen in Zugelementen. Dazu wird zwischen unterschiedlichen Steuerarten für einen Bowdenzugmotor gewählt. Dies erfolgt in Abhängigkeit einer Differenz von einer vorgegebenen Ablenkung zu einer tatsächlichen Ablenkung des Endabschnitts. Als unterschiedliche Steuerarten werden eine gepulste Ansteuerung und eine kontinuierliche Steuerung des Bowdenzugmotors vorgeschlagen.

Die US 4,941,454 betrifft einen Einsatz eines Servomotors zum Ablenken eines Endabschnitts mittels Bowdenzügen. Um eine definierte mechanische Spannung in den Bowdenzügen gewährleisten zu können, sind an dem Servomotor Stellschrauben vorgesehen, durch die die Länge der Bowdenzüge zum Servomotor variiert werden können. Ferner kann eine Grundstellung des Servomotors justiert werden, um die Stellung des Endabschnitts zusätzlich korrigieren zu können.

Die US 7,134,993 betrifft eine Vorrichtung, die einer Spannungslosigkeit in Bowdenzügen bei Endoskopen entgegenwirkt. Zu diesem Zweck wird ein Aktuator - hier ein Elektromotor - im Gesamten relativ zu den Bowdenzügen verlagert.

Bei einem automatischen System bedient der Anwender üblicherweise einen Schalter, um einen Elektromotor - einen Aktuator - zu betätigen. Durch Betätigung des Schalters erfolgt aufgrund der Anlauftotzeit für eine gewisse Zeit keine sichtbare Ablenkung des Endabschnitts, bis das Ablenken abrupt einsetzt. Nach einem Abschalten des Aktuators wird der Endabschnitt durch den Nachlaufweg weiter ausgelenkt. Die fehlende Kraftrückkopplung, wie sie bei Einsatz des Handrads vorliegt, macht es für den Anwender von elektrisch ablenkbaren Endabschnitten noch schwerer, die gewünschte exakte Ablenkung einzustellen.

Es ist daher eine Aufgabe der vorliegenden Erfindung, ein Endoskop der eingangs genannten Art dahingehend weiterzubilden, dass der ablenkbare Endabschnitt aus allen Ablenkwinkeln heraus schnell und hochgenau in einen neuen Ablenkwinkel abgelenkt werden kann.

Erfindungsgemäß wird die Aufgabe dadurch gelöst, dass die Steuereinheit weiter dazu ausgebildet ist, den Aktuator zur Vermeidung eines Nachlaufwegs durch axiales Entspannen des Zugelements am Ende der Ablenkbewegung dann in einer hohen Geschwindigkeitsstufe zu betreiben, vermieden wird, wobei der Aktuator das Zugelement, für das Entspannen, gegen die Zugrichtung bewegt.

Es ergibt sich der Vorteil, dass eine Zugkraft des Zugelements zum Ende der Ablenkbewegung von dem Aktuator eingestellt wird. Da dies in der hohen Geschwindigkeitsstufe erfolgt, kann dadurch der Nachlaufweg vermindert oder ganz verhindert werden. Die Steuereinheit steuert dabei den Aktuator derart, dass er das Zugelement in der hohen Geschwindigkeitsstufe gegen die Zugrichtung verlagert. Dadurch erfolgt eine Anpassung der Zugkraft sehr schnell, was zu einer hohen Genauigkeit und schnellen Umsetzung der tatsächlich benötigten Zugkraft führt. Der mechanische Aktuator weist verschiedene Geschwindigkeitsstufen auf, wobei der Aktuator eine Anzahl hoher und eine Anzahl niedriger Geschwindigkeitsstufen besitzt. Die Anzahl niedriger Geschwindigkeitsstufen wird vorzugsweise zum eigentlichen Ablenken des Endabschnitts verwendet. Somit wird das Zugelement durch den Aktuator zunächst in einer niedrigen Geschwindigkeitsstufe betrieben, um die Ablenkbewegung des distalen Endabschnitts herbeizuführen. Anschließend erfolgt eine Bewegung des Zugelements in Gegenrichtung, also gegen die Zugrichtung, in der hohen Geschwindigkeit, um das Zugelement schnell zu entspannen. Die Zugrichtung ist dabei diejenige Richtung, in die das Zugelement bewegt wird, während der Endabschnitt abgelenkt wird. Aufgrund der hohen Geschwindigkeitsstufe wird so erreicht, dass der Nachlaufweg vermindert oder verhindert werden kann. Das wird erreicht, indem das Zugelement sehr schnell entspannt wird, wodurch ein Nachlaufen nicht mehr erfolgen kann. Durch ausreichend schnelles Entspannen des Zugelements, und damit durch eine ausreichend hohe Geschwindigkeitsstufe, kann der Nachlaufweg bis auf Null reduziert werden. Während des axialen Entspannens des Zugelements verbleibt das Endstück so im Wesentlichen in seiner Momentanposition. Somit wird durch ein Vermindern des Nachlaufwegs eine hochgenaue Einstellung des Ablenkwinkels des Endabschnitts ermöglicht.

Der ablenkbare distale Endabschnitt ist ein Bereich des flexiblen Endoskopschafts, der in dem Endstück endet. Dieser Endabschnitt ist ablenkbar ausgebildet, damit das Endstück verschwenkt werden kann. Der proximale Endbereich des Endoskops ist typischerweise als Handgriff oder Endoskopgehäuse ausgebildet. Er enthält den mechanischen Aktuator, der das Zugelement verlagern kann. Ein derartiger mechanischer Aktuator kann als elektrische Maschine ausgebildet sein, die direkt oder über ein Getriebe eine Seilrolle antreibt. Die Seilrolle wirkt dann auf das Zugelement. Der Aktuator kann als Elektromotor, elektrischer Servomotor, Schrittmotor oder als Tauchspule ausgebildet sein.

Das Zugelement kann als Bowdenzug ausgebildet sein, welcher eine Bowdenzugseele enthält, die innerhalb einer Bowdenzugspirale geführt ist. In einigen Ausführungsbeispielen ist es vorgesehen, dass die Bowdenzugseele mittels eines Zugdrahts mit der Seilrolle gekuppelt ist. In diesem Fall bilden Bowdenzug und Zugdraht gemeinsam das Zugelement.

Die Steuereinheit kann zudem als integrierter Schaltkreis, wie beispielsweise als Mikroprozessor, ausgebildet sein. Eine Logik zur Steuerung der Geschwindigkeitsstufen kann Bestandteil dieses integrierten Schaltkreises sein. Sie kann aber auch als Softwareimplementierung in dem Mikroprozessor vorliegen. Die Steuerung der Geschwindigkeitsstufen wird vorzugsweise diskret vorgenommen, so dass zwischen unterschiedlichen, feststehenden Geschwindigkeitsstufen gewählt wird. Der Einsatz fester Geschwindigkeitsstufen ermöglicht eine sehr einfache Steuerung. Es ist aber auch denkbar, dass die Steuerung der Geschwindigkeitsstufen kontinuierlich erfolgt, so dass eine sehr präzise Steuerung der Geschwindigkeit und damit der Zugkraft möglich ist. Unter der hohen Geschwindigkeitsstufe wird eine Geschwindigkeitsstufe verstanden, die ein Mehrfaches der niedrigen Geschwindigkeitsstufe entspricht. Diese wird für das Ablenken des Endabschnitts selbst eingesetzt. Die niedrige Geschwindigkeitsstufe wird also dann verwendet, wenn sich der Endabschnitt tatsächlich bewegt.

In einer weiteren Ausgestaltung ist die Steuereinheit weiter dazu ausgebildet, den Aktuator dann in der hohen Geschwindigkeitsstufe zu betreiben, wenn zu Beginn der Ablenkbewegung eine Anlauftotzeit durch axiales Spannen des Zugelements besteht, wobei der Aktuator das Zugelement, für das Spannen, in die Zugrichtung bewegt.

In dieser Ausgestaltung ergibt sich der Vorteil, dass die Zugkraft des Zugelements zu Beginn der Ablenkbewegung von dem Aktuator eingestellt wird. Da dies in der hohen Geschwindigkeitsstufe erfolgt, kann dadurch die Anlauftotzeit vermindert oder ganz verhindert werden. Die Steuereinheit steuert dabei den Aktuator derart, dass er das Zugelement in der hohen Geschwindigkeitsstufe in die Zugrichtung verlagert. Dadurch erfolgt eine Anpassung der Zugkraft sehr schnell, was zu einer hohen Genauigkeit und schnellen Umsetzung der tatsächlich benötigten Zugkraft führt. Hierfür können die bereits beschriebenen Geschwindigkeitsstufen eingesetzt werden. Zunächst erfolgt eine Bewegung des Zugelements in der hohen Geschwindigkeit in Zugrichtung, um das Zugelement schnell zu spannen. Anschließen wird das Zugelement durch den Aktuator in einer niedrigen Geschwindigkeitsstufe betrieben, um die Ablenkbewegung des distalen Endabschnitts herbeizuführen. Die Zugrichtung ist dabei diejenige Richtung, in die das Zugelement während der Ablenkbewegung bewegt wird. In der hohen Geschwindigkeitsstufe wird die Anlauftotzeit minimiert, wodurch ein Anwender ein entsprechend schnelleres Ansprechen des Endabschnitts auf seinen Wunsch zum Ablenken hin erfährt. Während des axialen Spannens des Zugelements verbleibt das Endstück so im Wesentlichen in seiner Momentanposition. Durch Verminderung der Anlauftotzeit wird ein sehr schnelles Ablenken des Endabschnitts ermöglicht. Da eine Kompensation von Anlauftotzeit und Nachlaufweg durch variable Geschwindigkeitsstufen des Aktuators erfolgt, ergibt sich weiter der Vorteil, dass kein oder nur wenig zusätzlicher konstruktiver Aufwand betrieben werden muss, um diese Erfindung in Endoskopen umzusetzen.

In einer weiteren Ausgestaltung ist der Aktuator zu Beginn der Ablenkbewegung ein Spanner, indem der Aktuator das Zugelement um einen definierten Spannweg verlagert.

In dieser Ausgestaltung erfolgt das Spannen zeitlich direkt bevor der distale Endabschnitt abgelenkt wird. In bevorzugten Ausführungsbeispielen ist es vorgesehen, dass das Zugelement dann verlagert wird, wenn ein Anwender oder eine Steuerung ein Steuersignal vorgibt, um den distalen Endabschnitt abzulenken. Dabei wird das Feder-Dämpfer-System mit dem Aktuator bis kurz vor einen Punkt vorgespannt, ab dem sich der Endabschnitt bewegen würde, um anschließend abgelenkt zu werden. Zur Vorbereitung wird dadurch der Endoskopschaft gestaucht und das Zugelement gespannt, wobei das Endstück im Anschluss an das Spannen im Wesentlichen in die gleiche Richtung ausgerichtet ist wie zu dem Zeitpunkt, bevor das Zugelement um den definierten Spannweg verlagert wurde. Durch die hohe Geschwindigkeitsstufe wird erreicht, dass die Anfangstotzeit zwischen Beginn des Schaltsignals und Beginn des Ablenkens stark minimiert wird. Daraus ergibt sich der Vorteil, dass der Anwender die Anfangstotzeit nur wenig oder gar nicht wahrnimmt und damit das Ablenken des distalen Endabschnitts von Beginn an sehr genau steuern kann. Ab dem Punkt des Ablenkens wird die Geschwindigkeitsstufe des Aktuators gesenkt, und das Ablenken des Endabschnitts mit einer niedrigeren Geschwindigkeitsstufe beginnt.

Der Spannweg wird dadurch definiert, dass dem Aktuator der Spannweg von der Steuereinheit vorgegeben wird. Das heißt, das Definieren des Spannwegs erfolgt entweder, bevor das Zugelement um den Spannweg verlagert wird und/oder während das Zugelement verlagert wird, wie beispielsweise bei einer Regelung.

In einer weiteren Ausgestaltung ist der Aktuator zum Ende der Ablenkbewegung ein Entspanner, indem der Aktuator das Zugelement um einen definierten Entspannweg verlagert.

In dieser Ausgestaltung erfolgt die Verlagerung des Zugelements gegenläufig zu der Bewegung, die den Endabschnitt ausgelenkt hat. Um zu verhindern, dass der Nachlaufweg entsteht, wird das Zugelement zeitlich direkt nach Beenden des Ablenkens des Endabschnittes um den Entspannweg verlagert und dadurch entspannt. Das Verlagern des Zugelements erfolgt ebenfalls mit der hohen Geschwindigkeitsstufe des Spannens, oder mit einer anderen hohen Geschwindigkeitsstufe. Die hohe Geschwindigkeit bewirkt, dass der Nachlaufweg schnell und zuverlässig minimiert wird. Es ist insbesondere vorgesehen, dass der Aktuator dann das Zugelement um den Entspannweg verlagert, wenn das Schaltsignal eines Anwenders beendet ist. Das Ende des Schaltsignals kann so als Startsignal zum Verlagern des Aktuators eingesetzt werden. Daraus ergibt sich der Vorteil, dass der Anwender den Nachlaufweg nur wenig oder gar nicht wahrnimmt und damit das Ablenken des distalen Endabschnitts zum Ende des Ablenkens sehr genau steuern kann, ohne Nachlaufeffekte berücksichtigen zu müssen.

Der Entspannweg wird, wie der Spannweg, dadurch definiert, dass dem Aktuator der Entspannweg von der Steuereinheit vorgegeben wird. Das heißt, das Definieren des Entspannwegs erfolgt entweder, bevor das Zugelement um den Entspannweg verlagert wird, oder während das Zugelement verlagert wird, wie beispielsweise bei einer Regelung.

In einer weiteren Ausgestaltung weist das Endoskop eine Ermittlungseinheit auf, die den Spannweg oder den Entspannweg in Abhängigkeit mindestens eines Parameters ermittelt.

In dieser Ausgestaltung definiert die Ermittlungseinheit den Spannweg oder den Entspannweg. Dies erfolgt in Abhängigkeit von mindestens einem Parameter, was die Genauigkeit des Vor- und/oder Entspannens erhöht. Vorteilhaft ist hierbei, dass die Parameter sehr schnell und einfach an jedes einzelne Endoskop oder an Baureihen angepasst werden können. Der Weg - also der Spannweg oder der Entspannweg - hängt insbesondere von der zur Ablenkung des Endabschnitts benötigten Zugkraft ab. Die Zugkraft ist wiederum hauptsächlich vom aktuellen Ablenkwinkel des Endabschnitts und der Bauart des Endoskops abhängig. Beispielsweise ist typischerweise die benötigte Zugkraft um eine Geradeausstellung des Endabschnitts am geringsten. Die Geradeausstellung ist dabei die Stellung, in der der Endabschnitt im Wesentlichen gerade ausgerichtet ist. Bei einer vorhandenen Ablenkung des Endabschnitts, die sich kurz vor der maximal möglichen Ablenkung befindet, ist die benötigte Zugkraft am größten. Zudem hängt der benötigte Weg von der Richtung der zuvor ausgeführten Bewegungen ab. Wird beispielsweise die Ablenkung gestoppt und danach erneut der Endabschnitt in die gleiche Richtung weiter abgelenkt, dann wird ein anderer Weg benötigt als bei einer Umkehrung der Richtung des Ablenkens. Beide Wege können in bevorzugten Ausführungsbeispielen von der gleichen Ermittlungseinheit ermittelt werden.

Als Parameter eignet sich insbesondere der Ablenkwinkel des Endabschnitts. Dieser kann sowohl direkt als auch indirekt erfasst werden. Eine indirekte Erfassung ist in einfacher und wirtschaftlicher Weise umsetzbar. Für das indirekte Erfassen kann der Ablenkwinkel aus weiteren Parametern ermittelt werden. Als weitere Parameter sind hier eine Winkeldrehung eines Antriebsrades des Aktuators oder eine vorgegebene Laufzeit des Aktuators denkbar. Aus diesen Parametern kann der Ablenkwinkel beispielsweise durch Vergleich des Parameters mit einer geeigneten Vergleichswerttabelle oder durch Berechnung bestimmt werden. Derartige Vergleichswerttabellen enthalten vordefinierte Parameterlisten für die Bestimmung des Ablenkwinkels aus dem Parameter. Die Parameterlisten können in einem nicht flüchtigen Datenspeicher gespeichert sein, auf den die Steuereinheit Zugriff hat.

Ferner kann als weiterer Parameter ein elektrischer Strom verwendet werden, der den Aktuator versorgt.

In einer weiteren Ausgestaltung weist das Endoskop eine Messeinrichtung auf, die mindestens einen der Parameter erfasst.

In dieser Ausgestaltung misst die Messeinrichtung mindestens einen der Parameter. Das Messen des Parameters hat den Vorteil, dass der Parameter in seinem aktuellen, tatsächlich vorliegenden Betrag erfasst wird. Zudem bietet sich dadurch die Möglichkeit, dass die Steuerung einfach zu einer Regelung erweitert wird. Weiter ergibt sich der Vorteil, dass Alterserscheinungen direkt miterfasst und entsprechend berücksichtigt werden. Darunter fällt z.B. ein Verlängern des Zugelements aufgrund von Materialermüdung.

In einer weiteren Ausgestaltung weist die Messeinrichtung mindestens einen Sensor zur Messung der Position des Aktuators auf.

In dieser Ausgestaltung weist die Messeinrichtung mindestens einen Sensor auf, der die Position des Aktuators sensieren. Dies ist insbesondere dann vorteilhaft, wenn der Aktuator als Elektromotor ausgebildet ist und das Zugelement mittels eines Schnurrads (Seilrolle) verlagert wird. Als derartiger Sensor kommt insbesondere ein Halleffektsensor in Frage. Dieser misst berührungslos die Winkelstellung des Elektromotors oder des Schnurrads. Für den Halleffektsensor wird ein Magnet an einem Antriebsrad des Elektromotors oder an dem Schnurrad befestigt. Der Magnet arbeitet mit dem Halleffektsensor zusammen, wodurch die Winkelstellung sensiert wird. Anhand der aktuellen Winkelstellung kann der Weg beispielsweise mittels der Vergleichswerttabelle bestimmt werden.

In einer weiteren Ausgestaltung weist die Messeinrichtung mindestens einen Sensor zur Messung einer Zugkraft in dem Zugelement auf.

In dieser Ausgestaltung erfassen die Sensoren eine Zugkraft direkt innerhalb des Zugelements. Die Zugkraft ist einer der Parameter, der sehr einfach für die Ermittlung des Spannwegs oder des Entspannwegs verwendet werden kann. Durch den direkten Zusammenhang der Zugkraft mit den Wegen führt die Erfassung der Zugkraft zu einer besonders genauen Ermittlung des Spannwegs oder des Entspannwegs.

Zugelemente sowie flexible Endoskopschäfte altern während ihres Gebrauchs. Das kommt beispielsweise durch die hohe Beanspruchung in Autoklaven für eine Sterilisation und durch mechanische Spannungen in Betrieb der Endoskope zu Stande. Im Normalfall bedeutet diese Alterung, dass die Zugelemente während ihres Alterns gestreckt werden. Das führt dazu, dass die Ablenkung des Endabschnitts ein immer größeres Spiel bekommt, was eine hochgenaue Ablenkung erschwert. Um diesen Alterungsprozess zu kompensieren, ist es von Vorteil, die Zugkraft direkt zu erfassen. Vorteilhaft ist, dass der Alterungsprozess somit dauerhaft berücksichtigt wird. Eine mechanische neue Justierung der Zugelemente, beispielsweise durch einen Kundendienst, muss daher nicht oder zumindest nur selten vorgenommen werden. Ferner lässt sich mittels der Zugkraft auch der vom Aktuator aufgenommene Strom bestimmen. Es kann auch mittels dieser Sensoren zunächst ein Vorspannen des Zugelements erfolgen, dem das eigentliche Spannen durch Verlagerung des Zugelements um den Spannweg nachfolgt.

In einer weiteren Ausgestaltung ist eine Anzahl der Sensoren als Piezoelement ausgebildet.

In dieser Ausgestaltung wird die Zugkraft dadurch gemessen, dass mindestens einer der Sensoren ein Piezoelement ist, welches innerhalb des Zugelements angeordnet ist. Piezoelemente zeichnen sich dadurch aus, dass sie eine Kraftänderung einfach und sicher detektieren. Ferner sind Piezoelemente für industrielle Anwendungen verfügbar und damit wirtschaftlich einsetzbar. Ein Absolutwert für die Zugkraft selbst kann anhand eines Startwerts und der Zugkraftänderungen, die vom Piezoelement erfasst werden, rechnerisch ermittelt werden.

In einer weiteren Ausgestaltung ist eine Anzahl der Sensoren als Dehnungsmessstreifen ausgebildet.

In dieser Ausgestaltung wird die Zugkraft dadurch gemessen, dass mindestens einer der Sensoren als Dehnungsmessstreifen ausgebildet ist. Dies hat den Vorteil, dass er einen absoluten Wert für die Zugkraft erfasst. Dehnungsmessstreifen sind für industrielle Anwendungen verfügbar und somit wirtschaftlich einsetzbar. Sie erlauben eine direkte Erfassung des absoluten Werts in einem kontinuierlichen Messbereich. Somit kann die Zugkraft sehr exakt und kontinuierlich über Zeitspannen hinweg erfasst werden.

In einer weiteren Ausgestaltung ist mindestens einer der Sensoren als Strommesssensor ausgebildet, der einen elektrischen Strom zu dem Aktuator (32) erfasst.

In dieser Ausgestaltung wird die Zugkraft dadurch gemessen, dass mindestens einer der Sensoren als Strommesssensor ausgebildet ist. Dieser erfasst eine Stromaufnahme des mechanischen Aktuators, während dieser das Zugelement verlagert. Durch Ermittlung des Stroms kann die aufgebrachte Zugkraft auf das Zugelement ermittelt werden. Die Ermittlung der Zugkraft erfolgt hier vorzugsweise innerhalb der Ermittlungseinheit mittels einer Vergleichswerttabelle. Es handelt sich dabei um einen instrumentenspezifischen Parameter, der in dem Datenspeicher gespeichert ist. Ferner kann anhand dieses Parameters eine Begrenzung der Zugkraft durch Festlegung eines maximal zulässigen Wertes des elektrischen Stroms erfolgen. Dies ist besonders einfach mit einer elektronischen Regelung realisierbar. Auf diese Weise wird das Zugelement vor Überbeanspruchung und Zerstörung bewahrt.

Ein weiterer Faktor bei der Ermittlung des Spann- und/oder des Entspannwegs ist die Lage des Endoskopschafts. Diese lässt sich bei Verwendung des Endoskops nicht immer von außen ermitteln, beispielsweise wenn das Endoskop in einem Triebwerk liegt und dort eine Schleife bildet. In derartigen Fällen kann eine Ermittlung der Spann- und/oder Entspannwege alleine anhand der Aktuatorposition zu Messabweichungen führen. Durch Messung der Zugkraft in dem Zugelement wird eine Ermittlung des Spann- und/oder Entspannwegs unabhängig von der Lage des Endoskopschafts ermöglicht. Dabei wird vorzugsweise aus der aktuellen Aktuatorposition und dem an dieser Aktuatorposition benötigten elektrischen Strom ein Korrekturfaktor berechnet, der die Spann- und/oder Entspannwege im Vergleich zu denen des gerade ausgestreckten Endoskopschafts verändert. Die Verwendung kontinuierlicher Sensoren zur Messung der Zugkraft bietet hierbei den zusätzlichen Vorteil, dass sie nicht auf vereinzelte, fest definierte Kraftniveaus festgelegt sind. Dadurch kann das benötigte Kraftniveau für eine Änderung der Geschwindigkeitsstufen innerhalb eines Kraftbereichs frei gewählt werden. Beispielsweise kann so durch Anpassen von Parametern innerhalb der Steuereinheit die Ablenkung des distalen Endabschnitts unabhängig von der Lage des Endoskopschafts exakt eingestellt werden. Unter kontinuierlichen Sensoren zur Messung der Zugkraft in dem Zugelement werden hier insbesondere die vorgenannten Sensoren in Form von Piezoelementen, Dehnungsmessstreifen und/oder Strommesssensoren verstanden.

Bei der Ausgestaltung des Sensors als Strommesssensor kann während der normalen Ablenkbewegung, also während der distale Endabschnitt tatsächlich abgelenkt wird, eine kurz vor Ermittlung des Entspannwegs aufgewendete Aktuatorleistung gemessen werden. Mit dieser Aktuatorleistung ist das Zugelement - und damit das Federsystem vom Aktuator zum distalen Endabschnitt - vor Ermittlung des Entspannweges gespannt worden. Diese Aktuatorleistung entspricht einer bestimmten Zugkraft und damit einem bestimmten Federweg des Federsystems. Somit entspricht sie auch einer Stauchung des gesamten Endoskopschafts. Abhängig von dieser Zugkraft (und damit der Stauchung) wird mittels des Aktuators das Zugelement um den Spann- oder Entspannweg verlagert, wodurch die Stauchung kompensiert wird. Weiter können anhand dieser ermittelten Zugkraft die Spannwege ermittelt werden. Diese werden benötigt, um den distalen Endabschnitt in die jeweils möglichen Richtungen exakt zu verlagern. Insgesamt werden so die Spann- und Entspannwege unabhängig von der Lage des Schafts ermittelt. Liegt der Endoskopschaft während des Spannens und/oder Entspannens in einer Schleife, so werden die erforderlichen Kräfte seitens des Aktuators größer, was ebenfalls zu einer größeren Stauchung im Endoskopschaft führt. Gleichzeitig führt dies zu größeren ermittelten Spann- und Entspannwegen. In entsprechender Weise ist es möglich, die beschriebene Ermittlung von Spann- und Entspannwegen mittels kontinuierlich messenden Sensoren zur Messung der Zugkraft innerhalb des Zugelements durchzuführen. Eine auf dieser Vorgehensweise basierende Regelung funktioniert sehr gut, wobei sie unabhängig von der Lage des Endoskopschafts gleich gute Ergebnisse bei einer Kompensation der Anlauftotzeit und/oder des Nachlaufs erbringt.

In einer weiteren Ausgestaltung weist der Strommesssensor einen Spannungsmesssensor und einen Shuntwiderstand auf, wobei der Spannungsmesssensor eine elektrische Spannung am Shuntwiderstand erfasst.

Gemäß dieser Ausgestaltung befindet sich in einem Aktuatorstromkreis ein niederohmiger Shuntwiderstand. Ein Spannungsabfall an diesem Shuntwiderstand ist proportional zu der Stromaufnahme des Aktuators, also einem elektrischen Versorgungsstrom. Die elektrische Spannung am Shuntwiderstand wird von dem Spannungsmesssensor erfasst und vorzugsweise über einen AD-Wandler in die Steuereinheit eingelesen. Dort liegt der erfasste Wert für eine weitere Verarbeitung digital vor. Besonders vorteilhaft ist diese Art der Messung bei Verwendung eines Elektromotors als Aktuator. Jeder Elektromotor zeichnet sich dadurch aus, dass der aufgenommene Versorgungsstrom über eine sogenannte Drehmomentkonstante - eine individuelle Konstante, die von der Bauform des Elektromotors abhängt - direkt mit einem Abtriebsdrehmoment des Elektromotors auf dessen Motorwelle verknüpft ist. Sofern der Versorgungsstrom bekannt ist, lässt sich über die Drehmomentkonstante direkt das Abtriebsmoment berechnen, beispielsweise durch Division.

In bevorzugten Ausführungsbeispielen wirkt das Abtriebsdrehmoment des Elektromotors direkt auf das Schnurrad, das einen bestimmten Durchmesser besitzt. Eine Berechnung der Kraft, die hier auf den Umfang des Schnurrads und somit auf die zwei Seilzüge wirkt, erfolgt vorzugsweise, indem das Abtriebsdrehmoment durch den Radius des Schnurrads dividiert wird. Auf diese Weise lässt sich durch die Messung des Versorgungsstroms die Zugkraft ermitteln, die auf die Seilzüge wirkt.

In weiteren Ausführungsbeispielen kann es vorgesehen sein, dass zwischen dem Schnurrad und dem Aktuator ein Getriebe eingesetzt wird, welches einen Teil des vom Aktuator gelieferten Abtriebsdrehmoments durch innere Reibung verbraucht. In diesem Fall muss ein Getriebewirkungsgrad bei der Berechnung der Zugkraft berücksichtigt werden.

In einer weiteren Ausgestaltung ist eine Anzahl der Sensoren als Zugschalter ausgebildet.

In dieser Ausgestaltung wird die Zugkraft dadurch gemessen, dass mindestens einer der Sensoren als Zugschalter ausgebildet ist. Unter einem Zugschalter wird ein Bauelement verstanden, welches bei einer bestimmten aufgebrachten Zugkraft einen elektrischen Kreis schließt oder öffnet. Hierzu können Schließer als Zugschalter eingesetzt werden, welche den elektrischen Kreis dann schließen, wenn die bestimmte Zugkraft anliegt. Von der Ausbildung als Schließer wird im Folgenden ausgegangen. Über eine Feder in diesem Schließer wird die benötigte Kraft zum Schließen des Kontakts definiert. Dies kann dazu eingesetzt werden, dass der Aktuator so lange das Zugelement verlagert, bis der Zugschalter schließt, um damit den Spannweg zu definieren. So ist gewährleistet, dass beim Schließen des Zugschalters das Zugelement genau unter der Federkraft der Feder im Zugschalter steht. Umgekehrt ist es denkbar, dass der Aktuator das Zugelement so lange verlagert, bis das Schaltelement gerade öffnet, um den Entspannweg zu definieren. Durch die Verwendung mehrerer Zugschalter in mehreren Zugelementen wird eine entsprechend sichere Messung der Zugkraft ermöglicht. Insbesondere ist eine Verwendung von zwei Zugschaltern in zwei Zugelementen vorteilhaft, die gegensinnig zueinander arbeiten. Durch die Schaltstellungen der Zugschalter wird eine Auswertung der aktuellen Zugkraft in dem Zugelementenpaar und damit eine Bestimmung der Zugrichtung ermöglicht.

Bei Verwendung dieser diskreten Sensoren zur Messung der Zugkraft ist es ebenfalls möglich, wie oben beschrieben, eine Regelung der Auslenkung des distalen Endabschnitts zu realisieren. Auf Basis der Zugkraft im Zugelement, welche beim Schließen des Zugschalters ermittelt wird, kann der Spannweg und/oder der Entspannweg in entsprechender Weise ermittelt werden, wie dies oben mit Bezug zu kontinuierlichen Sensoren beschrieben ist. Es ist insbesondere vorgesehen, dass diese Art der Regelung alternativ oder ergänzend zu einer Regelung auf Basis einer Messung der Position des Aktuators erfolgt.

In einer weiteren Ausgestaltung besitzt mindestens einer der Sensoren einen ersten ohmschen Widerstand, dessen Widerstandswert dem Sensor eindeutig zugeordnet ist.

In dieser Ausgestaltung ist der Sensor individualisiert. Er besitzt einen individuellen elektrischen Widerstand, also einen Widerstand, dessen Widerstandswert dem Sensor eindeutig zugeordnet ist. Dies erfolgt zu dem Zweck, dass der Sensor von der Steuerungseinheit gegenüber anderen Sensoren unterschieden werden kann. Die Verwendung von zwei parallelgeschalteten Sensoren, jeweils einem in einem der Zugelemente, ermöglicht, zusätzlich die Zugrichtung zu ermitteln, in die das Zugelementepaar verlagert wird. Dies beinhaltet den Vorteil, dass eine sehr exakte und dynamische Auswertung ermöglicht wird. Auch ergibt sich die Möglichkeit einer Fehlererkennung.

In einer weiteren Ausgestaltung ist der Sensor als Zugschalter ausgebildet und besitzt den ersten ohmschen Widerstand für eine erste Schaltposition.

In dieser Ausgestaltung ist der Sensor als Zugschalter ausgebildet und mittels des ersten ohmschen Widerstands individualisiert. Wird ein erster ausreichender Betrag der Zugkraft auf den Zugschalter geführt, dann schließt der individualisierte Zugschalter. Aufgrund dessen erhält die Steuereinheit eine elektrische Spannung, deren Höhe einen Rückschluss darauf ermöglicht, welcher Zugschalter geschlossen ist. Zum Ablenken des Endabschnitts in einer Ablenkebene wird vorzugsweise ein Zugelementenpaar eingesetzt.

In einer weiteren Ausgestaltung besitzt der Zugschalter einen zweiten ohmschen Widerstand für eine zweite Schaltposition, wobei sich der Widerstandswert des zweiten Widerstandes von dem Widerstandswert des ersten Widerstandes unterscheidet.

In dieser Ausgestaltung besitzt der Zugschalter den ersten und einen zweiten ohmschen Widerstand. Dieser ist vorzugsweise derart innerhalb des Zugschalters angeordnet, dass er bei einem bestimmten zweiten Betrag der Zugkraft einen zweiten elektrischen Kreis schließt. Dieser zweite Betrag kann auch Null sein. Durch die Verwendung von zwei Widerständen können zwei unterschiedliche Schaltzustände bei zwei unterschiedlichen Beträgen unterschieden werden. Somit ist ein noch genaueres Definieren und damit eine dynamischere Regelung des Spannwegs oder Entspannwegs möglich. In einem bevorzugten Ausführungsbeispiel ist der dynamische Bereich der Feder zwischen zwei Beträgen der Zugkraft im Vorfeld definiert worden. Ein erster Stromkreis über den ersten Widerstand ist hier dann geschlossen, wenn die Zugkraft oberhalb des dynamischen Bereichs der Feder liegt. Ein zweiter Kontakt ist hier dann geschlossen, wenn die Zugkraft unter dem dynamischen Bereich liegt. Es ist ferner denkbar, eine Vielzahl von Widerständen innerhalb des Zugschalters einzusetzen, um eine Vielzahl von unterschiedlichen Beträgen erfassen zu können. Auch ist es denkbar, einen Widerstand einzusetzen, welcher sich kontinuierlich in seinem Widerstandwert bei Zugbeanspruchung entsprechend der Zugkraft verändert.

In einer weiteren Ausgestaltung bildet das Zugelement eine elektrische Leitung für den Sensor.

In dieser Ausgestaltung wird der Sensor über das Zugelement selbst bestromt. Dadurch ergibt sich der Vorteil, dass keinerlei Kabel zu und von dem Sensor geführt werden müssen. Auf diese Weise werden Bauraum und Bauteile eingespart. Weiter wird dadurch verhindert, dass Kabel abnutzen, die sich ständig mit den Zugelementen hin und her bewegen. Gleichzeitig wird eine Bruchgefahr derartiger Kabel reduziert. Somit wird durch diese Ausgestaltung eine Fehlersicherheit des Endoskops erhöht. Es kann vorgesehen sein, dass der Aktuator zusammen mit der Seilrolle und dem aktuatorseitigen Zugelement einen elektrischen Pol bildet und der Endoskopschaft mit dem Bowdenzug zum Endabschnitt einen anderen elektrischen Pol bildet. Diese beiden Pole sind galvanisch getrennt im Endoskopgehäuse angeordnet. Zwischen den beiden Polen befindet sich der Sensor.

In einer weiteren Ausgestaltung bildet das Zugelement eine Signalleitung für den Zugschalter.

In dieser Ausgestaltung wird der Sensor über das Zugelement selbst ausgewertet. Das Zugelement wird als Signalleitung eingesetzt und die vom Sensor erfassten Werte über die Signalleitung bspw. zu einer Steuereinheit oder Ermittlungseinheit übermittelt. Bei dem Einsatz des Zugelements als Signalleitung ergeben sich die bereits oben genannten Vorteile, dass Bauteile eingespart werden. Weiter wird dadurch verhindert, dass Signalleitungen abnutzen, die sich ständig mit den Zugelementen hin- und herbewegen. Somit wird auch durch diese Ausgestaltung eine Fehlersicherheit des Endoskops erhöht.

In einer weiteren Ausgestaltung ist der Aktuator verlagerbar zu der Messeinrichtung angeordnet und wirkt zur Messung der Zugkraft in dem Zugelement mit dem Sensor zusammen.

In dieser Ausgestaltung ist der Sensor außerhalb des Zugelements angeordnet. Es ist vorgesehen, dass der Aktuator aufgrund der Zugkraft verlagert werden kann. Dies erfolgt vorzugsweise relativ zu einem Gehäuse des Endoskops, insbesondere auch relativ zu dem Endoskopschaft. Die Verlagerung des Aktuators kann dabei translatorisch, beispielsweise in Richtung der Zugkraft, oder auch rotatorisch, durch Verkippen oder Verschwenken des Aktuators, erfolgen. Der entsprechende Sensor kann dabei als Druck- und/oder Zugsensor ausgebildet sein, gegen den der Aktuator beim Verlagern drückt oder an dem der Aktuator beim Verlagern zieht. Zudem sind unterschiedliche Arten von Sensoren denkbar, wie Kraftmesssensoren und/oder Wegmesssensoren. Ferner sind auch federgelagerte Aufhängungen für den Aktuator denkbar, wobei als Maß der Zugkraft eine Position des Aktuators gemessen wird. Vorteil ist eine einfache Anordnung des Sensors außerhalb des Zugelements. Dies ermöglicht eine einfachere und robustere Ausgestaltung der Messeinrichtung. Insbesondere ergibt sich eine einfache Wartung durch einfach und schnell austauschbare Sensoren.

In einer weiteren Ausgestaltung weist die Messeinrichtung mindestens einen Krümmungssensor zur Messung einer Krümmung des distalen Endabschnitts auf.

In dieser Ausgestaltung ist vorgesehen, dass die Krümmung des distalen Endabschnitts direkt gemessen wird. Dies erfolgt mittels des Krümmungssensors, der vorzugsweise im Bereich des distalen Endabschnitts angeordnet ist. Vorteilhaft hierbei ist eine besonders hohe Genauigkeit bei der Messung der Krümmung. Insbesondere wird dadurch ermöglicht, dass der absolute Wert der Krümmung direkt ermittelt wird, wobei Unsicherheiten einer indirekten Ermittlung der Krümmung vermieden werden.

In einer weiteren Ausgestaltung ist der Krümmungssensor ein Biegesensor.

In dieser Ausgestaltung wird als Krümmungssensor der Biegesensor eingesetzt. Der Biegesensor zeichnet sich dadurch aus, dass er die tatsächliche Krümmung direkt erfasst und somit hochgenau einsetzbar ist. Als Biegesensor sind beispielsweise piezoelektrische Biegesensoren oder widerstandsverändernde Biegesensoren denkbar.

In einer weiteren Ausgestaltung ist der Krümmungssensor ein Bewegungssensor.

In dieser Ausgestaltung ist es vorgesehen, dass als Krümmungssensor der Bewegungssensor eingesetzt wird. Unter einem Bewegungssensor werden insbesondere Geschwindigkeits- oder Beschleunigungssensoren verstanden. Vorteilhaft hierbei ist, dass diese Arten von Sensoren für industrielle Zwecke leicht verfügbar sind und mit geringen Bauräumen auskommen. Ferner existieren bereits kombinierte Beschleunigungssensoren, welche gleichzeitig die Beschleunigungen in mehrere räumliche Dimensionen erfassen. Mit diesen Sensoren kann eine Ermittlung der Bewegung des distalen Endabschnitts im gesamten erfolgt. Somit ergibt sich eine einfache, wirtschaftliche und hochgenaue Erfassung der Krümmung des distalen Endabschnitts.

Bei Verwendung des Krümmungssensors kann der Spannweg dadurch ermittelt werden, dass der Aktuator solange mit der hohen Geschwindigkeit aus dem Stillstand heraus betrieben wird, bis eine Signalveränderung am Krümmungssensor detektiert wird. Diese Signalveränderung deutet dann eine Ablenkung des distalen Endes. Sofort nach dem Detektieren dieser Signalveränderung wird der Aktuator mit der niedrigen Geschwindigkeit betrieben, wodurch der Anwender die kurzzeitig hohe Geschwindigkeit kaum wahrnehmen kann. Ebenso kann zur Ermittlung des Entspannwegs der Aktuator solange mit der hohen Geschwindigkeit betrieben werden, bis keine Signalveränderung am Krümmungssensor mehr detektierbar ist.

Dieses Vorgehen kann sowohl mittels des Biegesensors als auch in entsprechender Weise mittels des Bewegungssensors durchgeführt werden.

Besonders vorteilhaft ist eine Regelung der Krümmung des distalen Endabschnitts auf Basis mehrerer Parameter. Diese können in Form einer Kaskaden- oder einer Stufenregelung kombiniert werden. Unter einer Stufenregelung wird hier ein Regelkonzept verstanden, welches für bestimmte Ausgangssituationen bestimmte Regler einsetzt und die Regler nach entsprechenden Vorgaben während des Regelvorgangs auswechselt. Beispielsweise ist es denkbar, dass der elektrische Versorgungsstrom zum Aktuator ermittelt wird. Liegt dieser unterhalb eines gewissen Schwellwerts, wird die Regelung der Krümmung - und damit die Ermittlung des Spann- und/oder Entspannwegs - auf Basis der Position des Aktuators durchgeführt. Steigt der Versorgungsstrom jedoch über den Schwellwert, dann wird die Regelung auf Basis des Versorgungsstroms durchgeführt.

Es versteht sich, dass die vorstehend genannten und die nachstehend noch zu erläuternden Merkmale nicht nur in den angegebenen Kombinationen, sondern auch in anderen Kombinationen oder in Alleinstellung einsetzbar sind, ohne den Rahmen der vorliegenden Erfindung zu verlassen.

Die Erfindung wird nachfolgend anhand einiger ausgewählter Ausführungsbeispiele in Zusammenhang mit den beiliegenden Zeichnungen näher beschrieben und erläutert. Es zeigen:
- Figur 1: ein erstes Ausführungsbeispiel eines flexiblen Endoskops mit einem ablenkbaren Endabschnitt,
- Figur 2: das Endoskop von Figur 1 in schematischer teilweiser Schnittdar6stellung,
- Figur 3: schematisch den Schaft von Figur 1 und 2 in einer Schnittdarstellung, ohne Spannung eines Zugelements,
- Figur 4: den Schaft von Figur 3, wobei das Zugelement gespannt ist,
- Figur 5: den Schaft von Figur 3 und 4 mit einem ausgelenkten Endabschnitt,
- Figur 6: den Schaft von Figur 3 mit nachgelaufenem Endabschnitt ,
- Figur 7: einen vergrößerten Ausschnitt von Figur 2 sowie einen Teil des Endoskopschafts,
- Figur 8: den Ausschnitt von Figur 7, wobei ein Zugelement um einen Spannweg verlagert ist,
- Figur 9: den Ausschnitt von Figur 7, wobei der Endabschnitt abgelenkt ist,
- Figur 10: den Ausschnitt von Figur 7, wobei das Zugelement um einen Entspannweg verlagert ist,
- Figur 11: ein zweites Ausführungsbeispiel eines Endoskops, wobei zwei Zugschalter vorgesehen sind,
- Figur 12: einen elektrischen Ersatzschaltplan für das Endoskop von Figur 11,
- Figur 13: einen Zugschalter in einer ersten Ausführungsform mit einem ersten ohmschen Widerstand,
- Figur 14: einen Zugschalter in einer zweiten Ausführungsform mit einem ersten und einem zweiten ohmschen Widerstand,
- Figur 15: ein drittes Ausführungsbeispiel eines Endoskops, wobei zwei Piezoelemente vorgesehen sind,
- Figur 16: ein viertes Ausführungsbeispiel eines Endoskops, wobei zwei Dehnungsmessstreifen vorgesehen sind,
- Figur 17: ein fünftes Ausführungsbeispiel eines Endoskops, wobei ein Strommesssensor vorgesehen ist,
- Figur 18: ein sechstes Ausführungsbeispiel eines Endoskops, wobei ein Strommesssensor in Verbindung mit einem A/D-Wandler vorgesehen ist,
- Figur 19: ein erstes Ausführungsbeispiel eines verlagerbaren Aktuators, wobei der Aktuator translatorisch verlagerbar ist,
- Figur 20: ein zweites Ausführungsbeispiel eines verlagerbaren Aktuators, wobei der Aktuator rotatorisch verlagerbar ist und
- Figur 21: ein Ausführungsbeispiel eines distalen Endabschnitts, das einen Bewegungssensor aufweist.

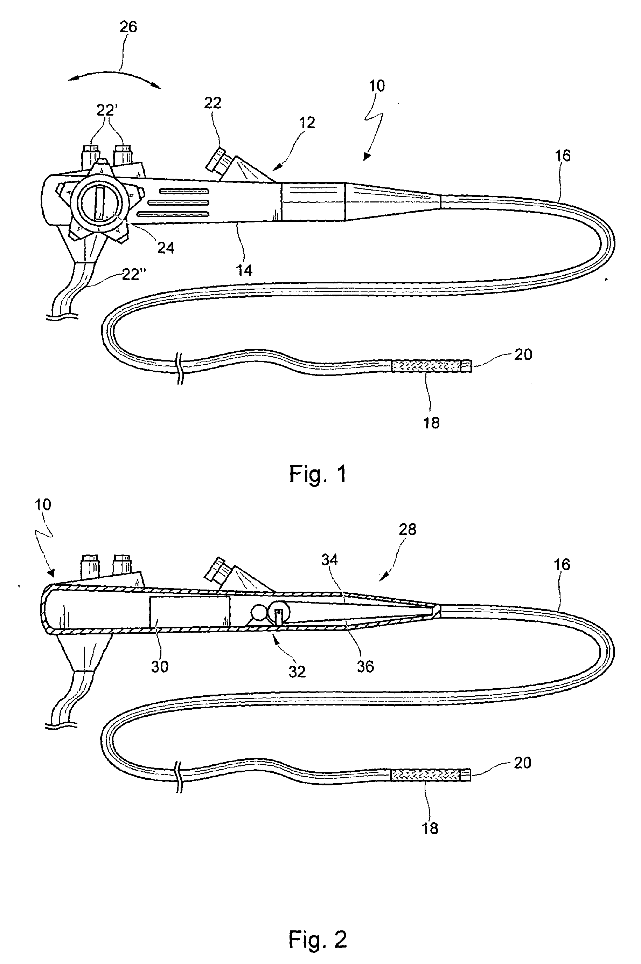

Das in den Figuren 1 bis 10 gezeigte Endoskop eines ersten Ausführungsbeispiels ist in seiner Gesamtheit mit der Bezugsziffer 10 versehen.

Das Endoskop 10 weist ein Kopfstück 12 mit einem Endoskopgehäuse 14 und einen flexiblen Endoskopschaft 16 auf. Das Endoskop 10 wird zu Untersuchungs- und/oder Operationszwecken in medizinischen Verfahren verwendet. In dem Endoskopschaft 16 verlaufen eine nicht dargestellte Endoskopoptik in Form von Lichtleitfasern, Bildleitern, verschiedene Kanäle, wie ein Saug- und ein Spülkanal, und ein Instrumentenkanal. Der Endoskopschaft 16 ist proximal mit dem Endoskopgehäuse 14 verbunden und erstreckt sich distal bis in einen distalen Endabschnitt 18. Dieser weist ein Endstück 20 in Form einer Abschlussbuchse auf. Das Endstück 20 ist der Bereich des Endoskopschafts 16, in dem die Lichtleitfasern, die Bildleiter und Kanäle enden. Der Endoskopschaft 16 ist in den Figuren 1 bis 10 nur teilweise dargestellt. Das Kopfstück 12 weist mehrere Anschlüsse 22, 22' und 22" auf. Der Anschluss 22 führt zu dem Instrumentenkanal. Durch ihn können Instrumente in den Endoskopschaft 16 und bis durch das Endstück 20 hindurch geführt werden. Dadurch können im Bereich vor dem Endstück 20 beispielsweise Operationen durchgeführt werden. Der Anschluss 22' ist ein Versorgungskabel, welches einen allgemeinen Versorgungszugang zum Endoskop 10 darstellt. Das Versorgungskabel beinhaltet verschiedene Arten von Versorgungen, wie beispielsweise eine elektrische Versorgung und Datenleitungen. Die Bildleitung des Endoskops 10 erfolgt über eine nicht dargestellte Kamera im Inneren des Endoskopgehäuses 14, welche Bilddaten über das Versorgungskabel nach außen leitet. Auf dem Endoskopgehäuse 14 sind Anschlüsse 22" in Form von zwei Leitungsstutzen vorgesehen, welche zu den Saug- und Spülkanälen führen. Für ein Steuern des Ablenkens des Endabschnitts 18 ist ein Drehschalter 24 vorgesehen, der in die Richtungen des Doppelpfeils 26 gedreht werden kann. Durch Drehen des Drehschalters 24 wird der ablenkbare Endabschnitt 18 in eine Richtung abgelenkt. Dies erfolgt entsprechend der Drehrichtung des Drehschalters 24 gegen oder in den Uhrzeigersinn.

Wie in Figur 2 dargestellt ist, ist in einem proximalen Endbereich 28 des Endoskopschafts 16 eine Steuereinheit 30 sowie ein Aktuator 32 angeordnet. Der proximale Endbereich 28 erstreckt sich in das Endoskopgehäuse 14 hinein. Der Aktuator 32 ist mit einem ersten Zugelement 34 und einem zweiten Zugelement 36 verbunden, die durch den Endoskopschaft 16 hindurchgeführt sind und sich bis in den Endabschnitt 18 hinein erstrecken. Die beiden Zugelemente 34 und 36 sind dabei einstückig ausgebildet.

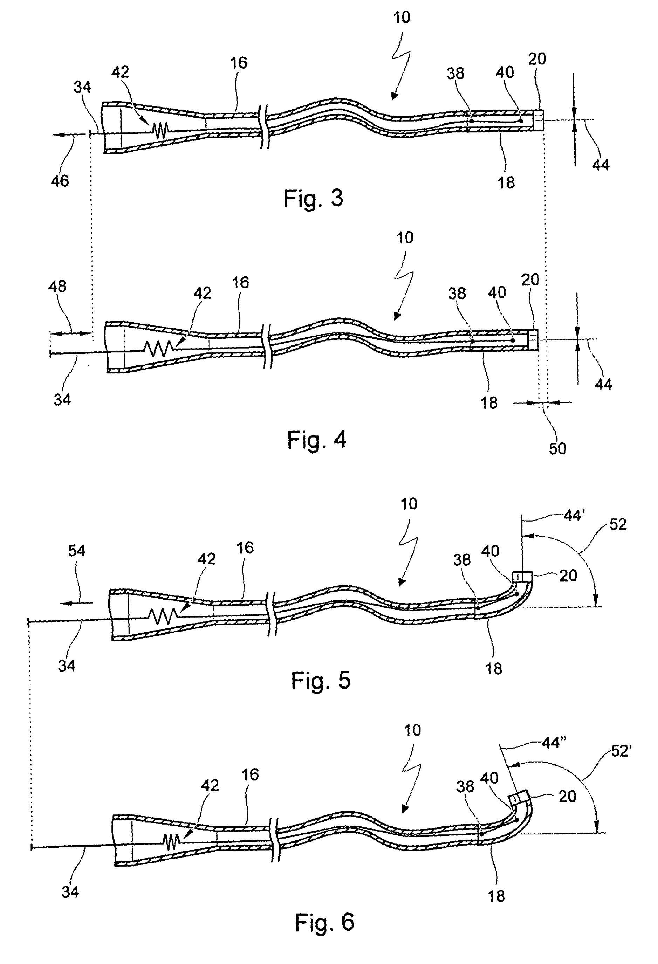

Figur 3 zeigt den Endabschnitt 18 in einer Geradeausstellung. Das erste Zugelement 34 ist entspannt innerhalb des Endoskopschafts 16 angeordnet. Es wird durch einen schematisch dargestellten Führungspunkt 38 im Endabschnitt 18 geführt. Anschließend ist das erste Zugelement 34 in einem Fixierpunkt 40 mit dem Endabschnitt 18 fest verbunden. Innerhalb des Zugelements 34 ist ein Federersatzelement 42 gezeigt, welches die gesamte Federwirkung des Zugelements 34 und des Endoskopschafts 16 symbolisiert. Das Zugelement 34 ist hier als Bowdenzug ausgebildet. Der Bowdenzug besteht aus einer Bowdenzugseele sowie einer Bowdenzugspirale, innerhalb der die Bowdenzugseele geführt ist. Der Führungspunkt 38 dient hier als schematischer Ersatz für die Bowdenzugspirale. Der Endabschnitt 18 sowie das Endstück 20 sind in einer Momentanposition 44 ausgerichtet, die der Geradeausstellung entspricht. Für einen Übergang von Figur 3 zu Figur 4 wird das Zugelement 34 in Richtung eines Pfeils 46 verlagert. Die Richtung gemäß des Pfeils 46 ist somit eine Zugrichtung.

In Figur 4 ist das Zugelement 34 in Richtung des Pfeils 46 um einen Weg 48 verlagert dargestellt. Durch diese Verlagerung des Zugelements 34 ist das Zugelement 34 gespannt. Daraus ergibt sich, dass die Lage des Zugelements 34 innerhalb des Endoskopschafts 16 so ist, dass das Zugelement 34 vom Fixierpunkt 40 zu seinem proximalen Ende den kürzesten Weg innerhalb des Endoskopschafts 16 einnimmt. Zudem ist das Federelement 42 hier gespannt. Weiter ist der Endoskopschaft 16 insbesondere im Bereich des Endabschnitts 18 durch die mechanische Spannung innerhalb des Zugelements 34 um einen Weg 50 verkürzt. Der Weg 48 des Zugelements 34 ist so definiert, dass das Zugelement 34 vorgespannt ist, wobei der Endabschnitt 18 im Wesentlichen noch in der Momentanposition 44 aus Figur 3 verbleibt. Ein weiteres Verlagern des Zugelements 34 in die gleiche Richtung wie Pfeil 46, würde zu einem Ablenken des Endabschnitts 18 führen. Der Weg 48 ist der Anlaufweg, die dafür benötigte Zeit die Anlauftotzeit.

Der Weg 48 muss immer für ein gewünschtes Ablenken des Endabschnitts 18 überwunden werden, bevor ein Ablenken möglich ist. Bei dem Endoskop 10 mit dem elektrischen Aktuator 32 führt der Weg 48 dazu, dass bei einem Einschalten des Aktuators 32 zunächst der Aktuator 32 das Zugelement 34 verlagert, ohne das der Endabschnitt 18 abgelenkt wird. Während der Zeit, in der der Aktuator 34 verlagert wird, der Anlauftotzeit, erkennt der Anwender nicht, ob überhaupt eine Reaktion auf seinen Wunsch erfolgt.

In Figur 5 ist der Endabschnitt 18 um einen Ablenkwinkel 52 abgelenkt. Durch den Ablenkwinkel 52 ergibt sich eine neue Momentanposition 44'. Die Momentanposition 44' stellt hier beispielhaft eine von dem Anwender des Endoskops 10 gewünschte neue Position des Endstücks 20 dar. Bedient der Anwender den Drehschalter 24 für ein Ablenken des Endabschnitts 18 und erreicht dabei das Endstück 20 die neue Momentanposition 44', so bringt der Anwender den Drehschalter 24 in eine neutrale Position um die Ablenkbewegung zu stoppen. Nun erwartet der Anwender, dass die Momentanposition 44' durch das Endstück 20 beibehalten wird. Die im gespannten Federersatzelement 42 noch enthaltene Energie, eine Federspannung, wird jedoch allmählich durch Entspannen des Federersatzelements 42 abgeleitet. Dadurch zieht sich das Zugelement 34 zusammen, und der Endoskopschaft 16 dehnt sich aus. Somit ergibt sich ein weiteres, relatives Verkürzen des Zugelements 34 zu dem Endoskopschaft 16. Dies entspricht einem weiteren Bewegen des Zugelements in Zugrichtung, entlang eines Pfeils 54. Folglich wird ein Abschnitt des Zugelements 34 zwischen dem Führungspunkt 38 und dem Fixierpunkt 40 noch weiter verkürzt, wodurch der Endabschnitt 18 weiter abgelenkt wird, wie dies in Figur 6 dargestellt ist. Der Endabschnitt 18 nimmt so den Ablenkwinkel 52' und die Momentanposition 44" ein. Auf diese Weise ergibt sich ein Nachlaufweg für das Endstück 20.

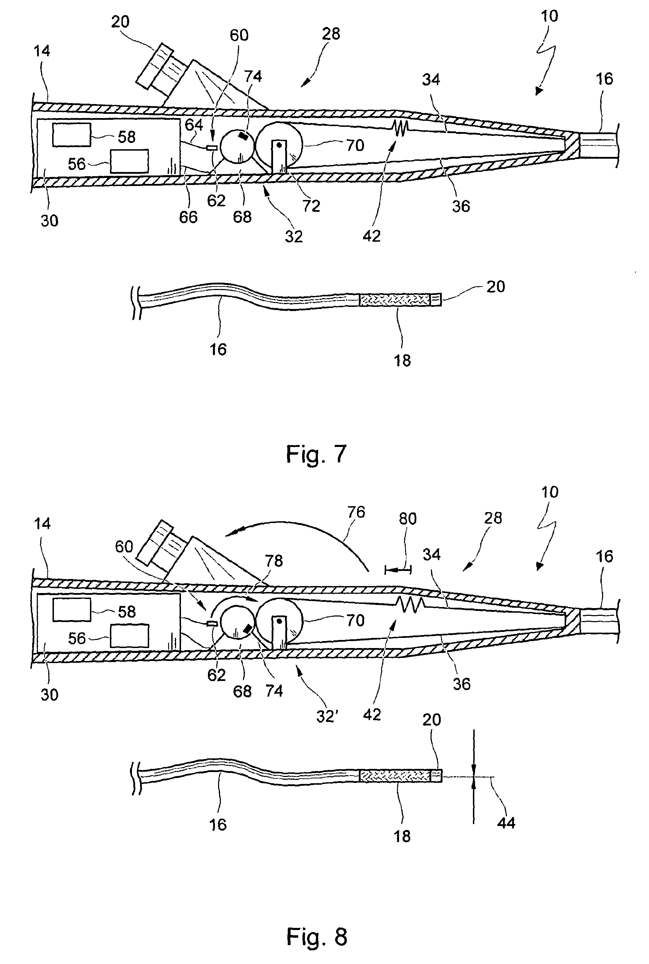

Figur 7 zeigt den ablenkbaren Endabschnitt 18 in der Geradeausstellung, wobei das Federersatzelement 42 und damit das gesamte Zugelement 34 entspannt ist. Es ist hier beispielhaft vorgesehen, dass der Anwender ausgehend von dieser Position den Endabschnitt 18 im Gegenuhrzeigersinn um 90° ablenken möchte. Die Steuereinheit 30 weist eine Ermittlungseinheit 56 auf, die ein integraler Bestandteil der Steuereinheit 30 ist. Ferner weist die Steuereinheit 30 einen nicht flüchtigen Datenspeicher 58 auf, der ebenso in die Steuereinheit 30 integriert ist. Die Steuereinheit 30 ist mit einer Messeinrichtung 60 verbunden, die einen Halleffektsensor 62 besitzt. Der Halleffektsensor 62 ist mittels einer Leitung 64 mit der Steuereinheit 30 verbunden. Zudem ist die Steuereinheit 30 über eine Leitung 66 mit einem Elektromotor 68 verbunden, der Teil des Aktuators 32 ist. Der Elektromotor 68 treibt eine Seilrolle 70 an, die in einer Lagerung 72 drehbar gelagert ist. Am Elektromotor 68 ist ein Magnet 74 befestigt, so dass er sich mitsamt einem Antriebsrad des Elektromotors 68 bewegt. Der Magnet 74 ist ebenfalls Teil der Messeinrichtung 60 und wirkt mit dem Halleffektsensor 62 zusammen. Dieser erfasst einen Drehwinkel des Elektromotors 68 als Parameter.

In dem nicht flüchtigen Datenspeicher 58 ist eine Vergleichswerttabelle mit Vergleichswerten zu dem Parameter gespeichert. In der Ermittlungseinheit 56 wird mit Hilfe der Vergleichswerttabelle ein Ablenkwinkel des Endabschnitts 18 ermittelt. Derartige Vergleichswerttabellen können individuell für jedes Gerät oder vor einer Herstellung für ganze Baureihen ermittelt, festgelegt und in dem nicht flüchtigen Datenspeicher 58 abgespeichert werden. Die Steuereinheit 30 steuert den Elektromotor 68 derart, dass die gewünschte Zugkraft im Zugelement 34 zustande kommt, die dann zunächst das Federersatzelement 42 spannt. Um dies zu erreichen, wird anhand des Ablenkwinkels 52 der benötigte Spannweg ermittelt. Dies kann ebenfalls mittels einer geeigneten Vergleichswerttabelle erfolgen. Die Steuereinheit 30 besitzt weiter eine Maximalbegrenzung, die anhand eines erfassten Stroms, der den Elektromotor 68 speist, überwacht, ob die Zugelemente 34 und 36 überbeansprucht werden. So wird ein Abreißen der Zugelemente 34 und 36 verhindert.

Figur 8 zeigt ein Verlagern des ersten Zugelements 34, wobei die Seilrolle 70 mit hoher Geschwindigkeit in Richtung eines Pfeils 76, also in Zugrichtung, gedreht wird. Dies erfolgt, nachdem der Anwender den Drehschalter 24 gegen den Uhrzeigersinn betätigt hat und damit ein Schaltsignal erzeugt. Das Schaltsignal wird von der Steuereinheit 30 empfangen. Aufgrund des Schaltsignals wird dann die Winkelposition des Antriebs 32 erfasst und an die Steuereinheit 30 übergeben. In der Steuereinheit 32 wird die Winkelposition in der Ermittlungseinheit 56 mit der Vergleichswerttabelle verglichen, aus der entweder direkt der Spannweg oder ein aktueller Ablenkwinkel ermittelt wird. Wird der Ablenkwinkel ermittelt, so erfolgt die Bestimmung des Spannwegs auf die gleiche Art mittels einer weiteren Vergleichswerttabelle. Der Aktuator 32 wird dann von der Steuereinheit 30 so lange betätigt, bis das Zugelement 34 in Zugrichtung um den Spannweg 80 verlagert ist, wodurch er einen Spanner 32' bildet. Hierfür dreht der Elektromotor 68 in Richtung eines Pfeils 78 mit einer hohen Geschwindigkeitsstufe. Durch die Bewegung der Seilrolle 70 wird das Zugelement 34 um den Spannweg 80 verlagert. Dadurch spannt sich das Federersatzelement 42. Der Spannweg 80 ist so lang bemessen, dass der Endabschnitt 18 gerade noch in seiner Momentanposition 44 verbleibt.

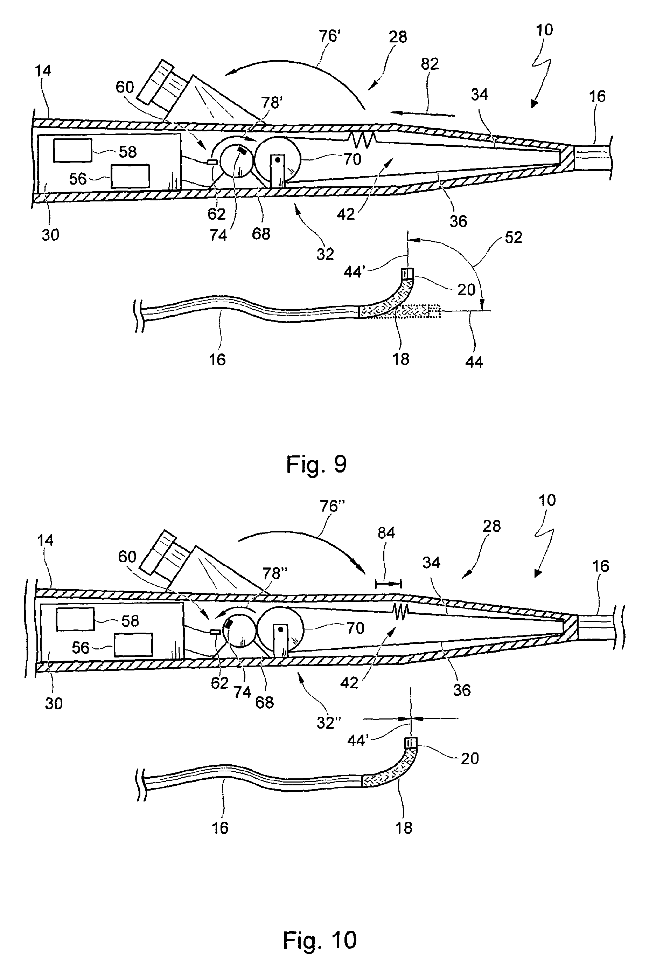

Figur 9 zeigt ein Ablenken des Endabschnitts 18 nach dem Spannen des Zugelements 34. Das Ablenken erfolgt durch weiteres Verdrehen der Seilrolle 70 in Richtung eines Pfeils 76' mit einer niedrigeren Geschwindigkeitsstufe. Durch das Verdrehen der Seilrolle 70 wird der Endabschnitt 18 aus seiner ursprünglichen Momentanposition 44 in seine neue Momentanposition 44' verlagert. Die neue Momentanposition 44' unterscheidet sich um einen Ablenkwinkel 52 von 90° von der ursprünglichen Momentanposition 44. Die neue Momentanposition 44' ist die vom Anwender gewünschte Position. Daher stellt der Anwender nun den Drehschalter 24 zurück in eine neutrale Stellung und beendet somit das Schaltsignal, um die Momentanposition 44' beizubehalten.

Figur 10 zeigt das Verringern des Nachlaufwegs direkt nach Beendigung des Schaltsignals. Der Nachlaufweg wird dadurch verringert, dass die Seilrolle 70 in Gegenrichtung zu den bisherigen Bewegungen und damit entgegen der Zugrichtung entlang eines Pfeils 76 gedreht wird. Dies erfolgt dadurch, dass der Elektromotor 68 von der Steuereinheit 30 in die entsprechende Richtung 78" gedreht wird. Somit verlagert das Zugelement 34 seine Position um einen Entspannweg 84, wodurch der Aktuator 32 einen Entspanner 32" bildet. Der Entspannweg 84 ist derart bemessen, dass das Federersatzelement 42 vollständig entspannt ist und keine Energie innerhalb des Federersatzelements 42 vorhanden ist, um den Endabschnitt 18 weiter zu verlagern. Somit verbleibt der Endabschnitt 18 in seiner Momentanposition 44", und das Nachlaufen ist verringert.

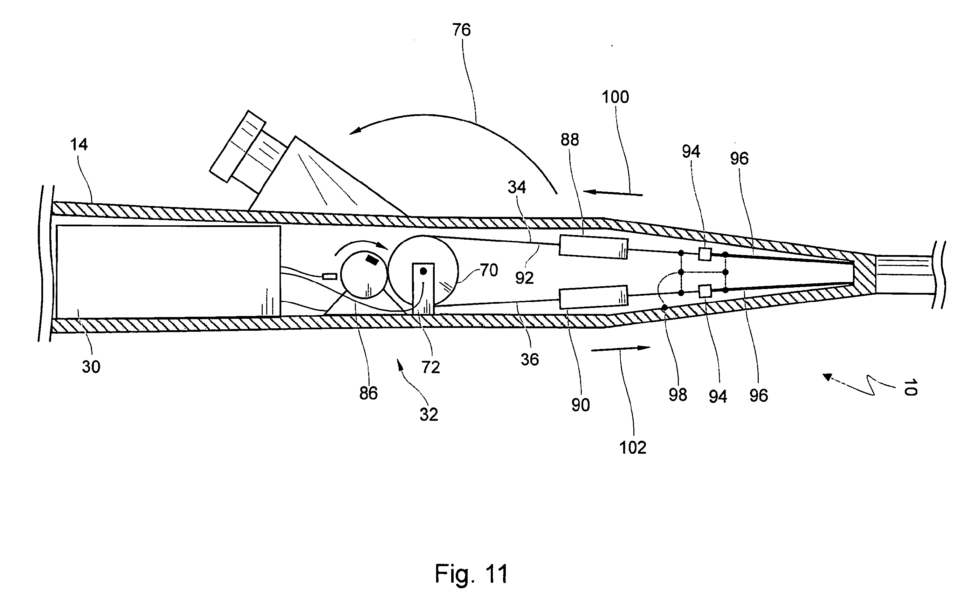

Figur 11 zeigt ein zweites Ausführungsbeispiel des Endoskops 10. Es ist eine Vergrößerung der Schnittdarstellung des Endoskopgehäuses 14 dargestellt. Innerhalb des Endoskopgehäuses 14 befindet sich die Steuereinheit 30, welche über eine Leitung 86 mit der Lagerung 72 elektrisch verbunden ist. Ferner ist die Steuereinheit 30 mit dem Endoskopgehäuse 14 als Masse elektrisch verbunden, was in der Figur nicht dargestellt ist. Weiter besteht eine elektrische Verbindung zwischen der Lagerung 72 und der Seilrolle 70. Die elektrische Verbindung setzt sich über das erste Zugelement 34 und das zweite Zugelement 36 zu den Zugschaltern 88 und 90 fort. Die Zugelemente 34 und 36 sind hier als Zugdraht 92 ausgebildet. Der Zugdraht 92 ist an den Zugschaltern 88 und 90 unterbrochen und erstreckt sich nach den Zugschaltern 88 und 90 weiter bis zu Bowdenzugterminierungen 94, welche in den Bowdenzug 96 selbst übergehen. Der Zugdraht 92 ist über strichpunktiert dargestellte Leitungen mit einem Masseanschluss 98 elektrisch verbunden. Die Leitungen von den Bowdenzügen 96 und dem Zugdraht 92 zum Masseanschluss 98 sind hier schematisch dargestellt. Durch Bewegen der Seilrolle 70 in Richtung des Pfeils 76 entsteht eine Zugkraft 100 innerhalb des Zugdrahts 92. Ferner entsteht eine Zugkraft 102. Durch die Zugkraft 100 wird der Zugschalter 88 ausgelenkt. Somit wird ein elektrischer Kreis ausgehend von der Steuereinheit 30 über den Zugdraht 92 und den Zugschalter 88 zum Masseanschluss 98 geschlossen. Durch Schließen dieses Kreises erhält die Steuereinheit 30 ein Signal, das von ihr interpretiert werden kann. Somit bilden die Zugelemente 34, 36 jeweils eine elektrische Leitung für die Sensoren, hier die Zugschalter 88 und 90. Ferner werden dadurch die Zugelemente 34, 36 auch als Signalleitung verwendet. Das Schließen des Zugschalters 88 bedeutet, dass die Zugkraft 100 mindestens einer Federkraft innerhalb des Zugschalters 88 entspricht. Auf diese Weise kann die Zugkraft innerhalb des Zugelements 34 bestimmt werden.

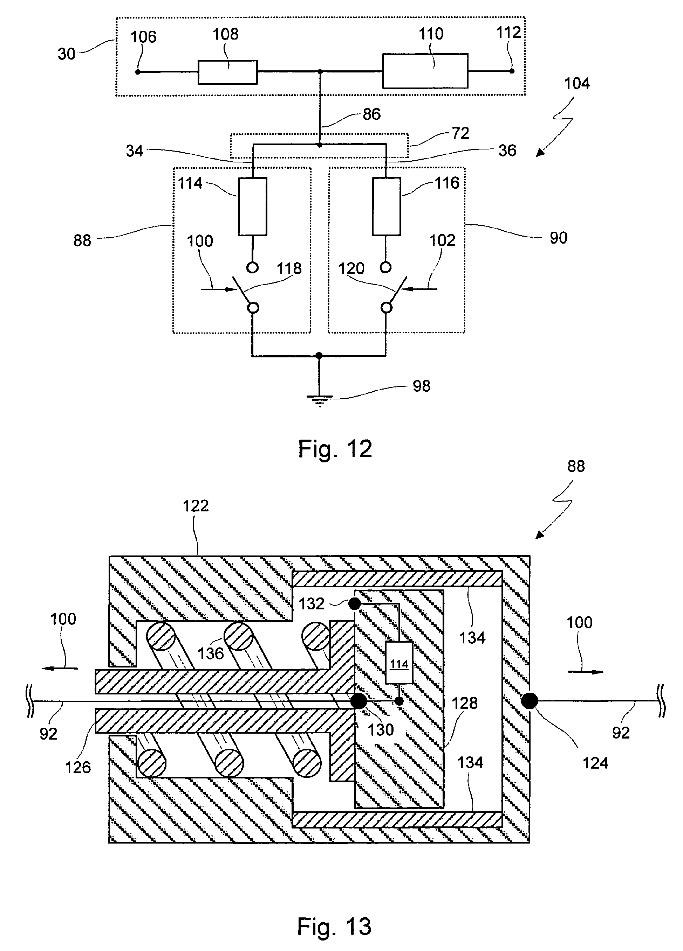

Figur 12 zeigt eine Ersatzschaltung 104. Die einzelnen Elemente aus Figur 11 sind hier schematisch mittels gestrichelter Linien dargestellt. Die Steuereinheit 30 umfasst einen Versorgungsanschluss 106, an welchem eine Versorgungsspannung angelegt wird. Der Versorgungsanschluss 106 ist mit einem Leitungswiderstand 108 verbunden, welcher einen ohmschen Widerstand der gesamten Leitungen darstellt. Der Leitungswiderstand 108 ist mit einem Knotenpunkt verbunden, von dem aus eine Leitung zu einem Komparator 110 führt. Der Komparator 110 führt ausgangsseitig zu einem Steuerungsanschluss 112. Dieser stellt innerhalb der Steuereinheit 30 die empfangene Information für eine weitere Bearbeitung bereit. Ausgehend von dem Knotenpunkt verläuft die Leitung 86 zu der Lagerung 72, von der aus sich der elektrische Kreis über die Zugelemente 34 und 36 auf die beiden Zugschalter 88 und 89 verzweigt. Der Zugschalter 88 enthält einen ersten ohmschen Widerstand 114, der zu einem Schaltelement 118 - hier ein Schließer - in Reihe geschaltet ist. Der Zugschalter 88 schließt dann, wenn die Zugkraft 100 ausreichend groß ist. Der Zugschalter 90 ist in gleicher Weise aufgebaut wie der Zugschalter 88 und enthält einen zweiten ohmschen Widerstand 116. Der Widerstand 116 hat einen anderen Widerstandswert als der Widerstand 114 und ist in Reihe zu einem Schaltelement 120 geschaltet. Das Schalterelement 120 schließt dann, wenn die Zugkraft 102 auf das Schalterelement 120 ausreichend groß ist. Beide Zugschalter 88 und 90 sind ausgangsseitig mit der Masse 98 verbunden.

Wenn eine ausreichende Zugkraft 100 oder 102 auf die Schalter 88 oder 90 wirkt, dann wird der elektrische Kreis geschlossen, der ein Signal am Steuerungsanschluss 112 ausgibt. Die elektrische Spannung des Signals ist dabei abhängig von den individuellen Widerstandswerten der entsprechend zugeschalteten Widerstände 114 oder 116. Somit kann die Steuereinheit 30 erkennen, welcher Zugschalter 88 oder 90 geschlossen wurde.

Figur 13 zeigt eine Schnittdarstellung des Zugschalters 88 in einem ersten Ausführungsbeispiel. Der Zugschalter 88 besitzt ein Schaltergehäuse 122, welches aus elektrisch leitendem Material besteht. An das Schaltergehäuse 122 ist mit einer Fixierung 124 der Zugdraht 92 befestigt. Auf der gegenüberliegenden Seite der Fixierung 124 ist ein zweiter Teil des Zugdrahts 92 mit dem Zugschalter 88 verbunden. Dieser ist durch einen Schaltzylinder 126 hindurchgeführt, welcher aus elektrisch isolierendem Material besteht. Der Zugdraht 92 ist nach seiner Durchführung durch den Schaltzylinder mittels einer Fixierung 130 mit einem Schaltstempel 128 elektrisch und mechanisch verbunden. Innerhalb des Schaltstempels 128 ist der erste Widerstand 114 angeordnet, welcher die Fixierung 130 elektrisch mit dem Kontakt 132 verbindet. Der Schaltstempel 128 ist gegenüber dem Schaltergehäuse 122 durch Isolation 134 isoliert. Mittels einer Feder 136 wird der Schaltzylinder 126 nebst dem Schaltstempel 128 in einer Ruhelage gehalten. Sie hält den Kontakt 132 davon ab, zwanglos mit dem Schaltergehäuse 122 elektrisch in Verbindung zu kommen. Durch Aufbringen der Zugkraft 100 an dem Zugdraht 92 wird erreicht, dass der Schaltstempel 128 aus seiner Ruhelage verlagert wird. Ist die Zugkraft 100 ausreichend groß, so wird die Feder 136 soweit komprimiert, dass der Kontakt 132 elektrisch mit dem Gehäuse 122 verbunden wird. Es folgt, dass ein elektrischer Strom ausgehend von dem Kontakt 130 zur Fixierung 124 geleitet wird und somit der Zugschalter 88 geschlossen ist.

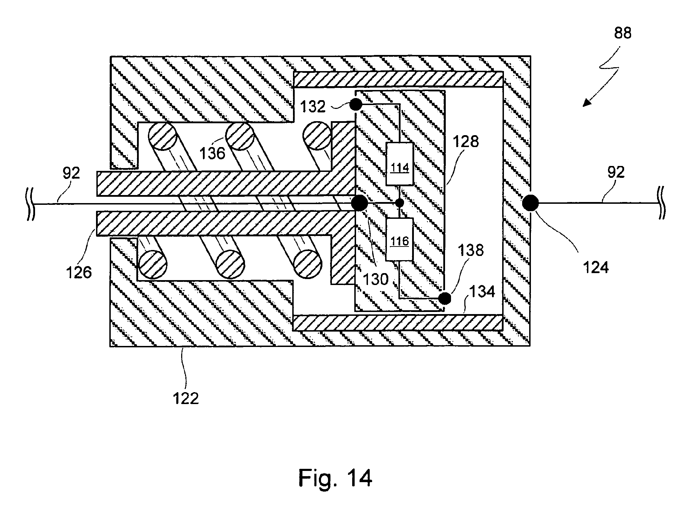

Figur 14 zeigt ein zweites Ausführungsbeispiel des Zugschalters 88 mit zwei Widerständen 114 und 116. Die Struktur des Zugschalters 88 unterscheidet sich hier durch den Widerstand 116, welcher parallel zu dem Widerstand 114 in dem Schaltstempel 128 angeordnet ist. Der Widerstand 116 verbindet die Fixierung 130 mit einem weiteren Kontakt 138. Der Kontakt 138 ist dann mit dem Gehäuse 122 verbunden, wenn sich der Schaltstempel 126 in einer Ruhelage am Gehäuse 122 abstützt. Diese Ruhelage wird dann erreicht, wenn nur geringe oder keine Zugkraft am Zugdraht 92 anliegt, wodurch die Feder 136 den Schaltstempel 126 gegen das Gehäuse 122 presst. Somit ergibt sich eine erste Schaltstellung, die über den zweiten Widerstand 116 detektierbar ist. Liegt eine ausreichende Zugkraft an dem Zugdraht 92 an, ergibt sich eine zweite Schaltstellung, die über den ersten Widerstand 114 detektierbar ist.

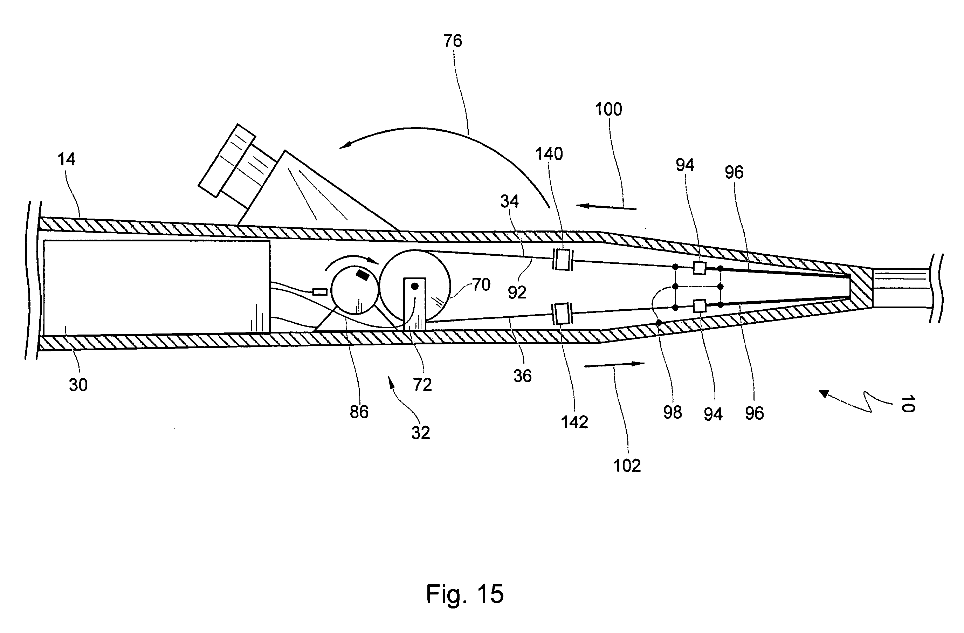

Figur 15 zeigt ein drittes Ausführungsbeispiel des Endoskops 10. Es ist eine Vergrößerung der Schnittdarstellung des Endoskopgehäuses 14 dargestellt. Innerhalb des Endoskopgehäuses 14 befindet sich die Steuereinheit 30, welche über die Leitung 86 mit der Lagerung 72 elektrisch verbunden ist. Ferner ist die Steuereinheit 30 mit dem Endoskopgehäuse 14 als Masse elektrisch verbunden, was in der Figur nicht dargestellt ist. Weiter besteht eine elektrische Verbindung zwischen der Lagerung 72 und der Seilrolle 70. Die elektrische Verbindung setzt sich über das erste Zugelement 34 und das zweite Zugelement 36 zu Piezoelementen 140 und 142 fort. Die Zugelemente 34 und 36 sind hier als Zugdraht 92 ausgebildet. Der Zugdraht 92 ist an den Piezoelementen 140 und 142 unterbrochen. Er erstreckt sich nach den Piezoelementen 140 und 142 weiter bis zu Bowdenzugterminierungen 94, welche in den Bowdenzug 96 selbst übergehen. Der Zugdraht 92 ist über strichpunktiert dargestellte Leitungen mit einem Masseanschluss 98 elektrisch verbunden. Die Leitungen von den Bowdenzügen 96 und dem Zugdraht 92 zum Masseanschluss 98 sind hier schematisch dargestellt. Durch Bewegen der Seilrolle 70 in Richtung des Pfeils 76 entsteht die Zugkraft 100 innerhalb des Zugdrahts 92. Ferner entsteht die Zugkraft 102. Durch die Zugkraft 100 wird das Piezoelement 140 angeregt. Somit wird eine elektrische Ladung von dem Piezoelement 140 zu der Steuereinheit 30 geleitet. Auf diese Weise erhält die Steuereinheit 30 ein Signal über Zugkraftänderungen, das von ihr interpretiert werden kann. Das Zugelement 34 wird somit sowohl als elektrische Leitung als auch als Signalleitung verwendet. Durch rechnerische Integration der erfassten Zugkraftänderungen in der Steuereinheit 30 wird die Zugkraft innerhalb des Zugelements 34 bestimmt. Die Zugkraft 102 wirkt in entsprechender Weise mit dem Piezoelement 142 zusammen.

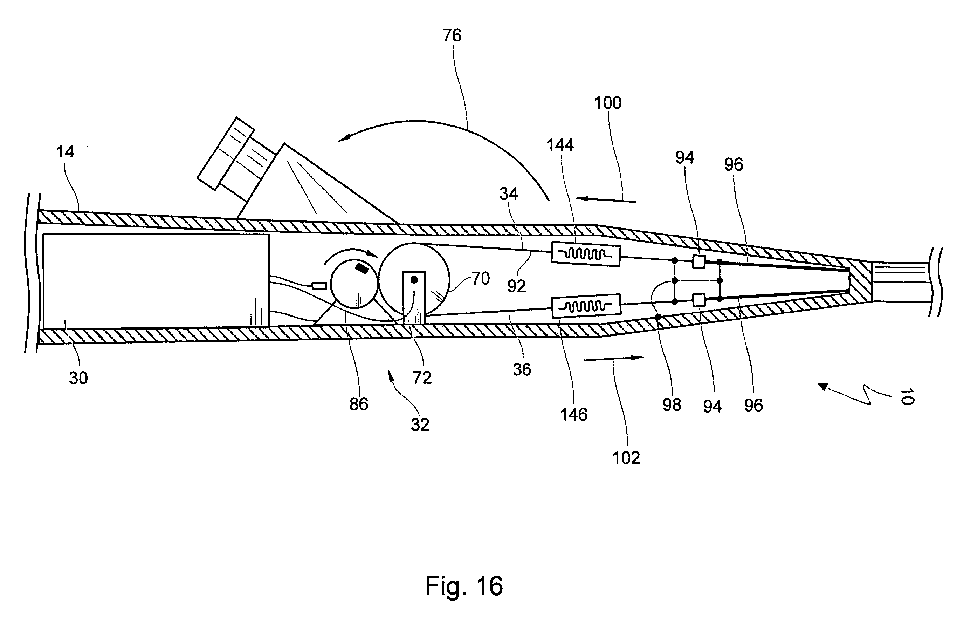

Figur 16 zeigt ein viertes Ausführungsbeispiel des Endoskops 10. Es ist eine Vergrößerung der Schnittdarstellung des Endoskopgehäuses 14 dargestellt. Innerhalb des Endoskopgehäuses 14 befindet sich die Steuereinheit 30, welche über die Leitung 86 mit der Lagerung 72 elektrisch verbunden ist. Ferner ist die Steuereinheit 30 mit dem Endoskopgehäuse 14 als Masse elektrisch verbunden, was in der Figur nicht dargestellt ist. Weiter besteht eine elektrische Verbindung zwischen der Lagerung 72 und der Seilrolle 70. Die elektrische Verbindung setzt sich über das erste Zugelement 34 und das zweite Zugelement 36 zu Dehnungsmessstreifen 144 und 146 fort. Die Zugelemente 34 und 36 sind hier als Zugdraht 92 ausgebildet. Der Zugdraht 92 ist mit den Dehnungsmessstreifen 144 und 146 verbunden und erstreckt sich zwischen den Bowdenzugterminierungen 94, welche in den Bowdenzug 96 selbst übergehen. Der Zugdraht 92 ist über strichpunktiert dargestellte Leitungen mit einem Masseanschluss 98 elektrisch verbunden. Die Leitungen von den Bowdenzügen 96 und dem Zugdraht 92 zum Masseanschluss 98 sind hier schematisch dargestellt. Durch Bewegen der Seilrolle 70 in Richtung des Pfeils 76 entsteht die Zugkraft 100 innerhalb des Zugdrahts 92 und ferner entsteht die Zugkraft 102. Durch die Zugkraft 100 wird der Dehnungsmessstreifen 144 gedehnt. Somit wird der Dehnungsmessstreifen in Abhängigkeit der Zugkraft verformt, wodurch dieser seinen elektrischen Widerstand verändert. Über den Zugdraht 92 kann der aktuelle Widerstand im Dehnungsmessstreifen 144 erfasst werden. Zu diesem Zweck muss der Zugdraht 92 zwischen Anschlüssen des Dehnungsmessstreifens 144 elektrisch unterbrochen sein, so dass der Zugdraht 92 als elektrische Leitung von und zu dem Dehnungsmessstreifen 144 eingesetzt werden kann. Dadurch wird der Zugdraht 92 als Signalleitung eingesetzt. Der Widerstandswert des Dehnungsmessstreifens 144 wird von der Steuereinheit 30 ausgewertet, wodurch die Zugkraft innerhalb des Zugelements 34 bestimmt wird. Die Zugkraft 102 wirkt in entsprechender Weise mit dem Dehnungsstreifen 146 zusammen.

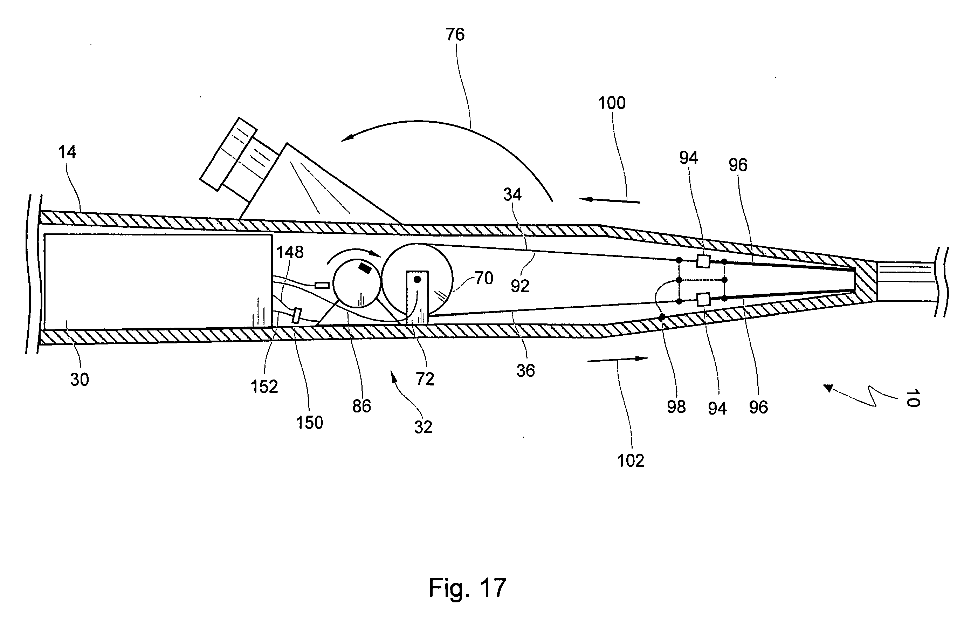

Figur 17 zeigt ein fünftes Ausführungsbeispiel des Endoskops 10. Es ist eine Vergrößerung der Schnittdarstellung des Endoskopgehäuses 14 dargestellt. Innerhalb des Endoskopgehäuses 14 befindet sich die Steuereinheit 30. Die Steuereinheit 30 ist über eine Leitung 148 mit einem Strommesssensor 150 elektrisch verbunden. Der Strommesssensor 150 misst einen elektrischen Strom in einer Leitung 152 zu dem mechanischen Aktuator 32, beispielsweise einen Versorgungsstrom. Der benötigte elektrische Strom verändert sich in Abhängigkeit der anliegenden Zugkraft, die auf den Zugdraht 92 wirkt. Mit anderen Worten, die mechanische Arbeit, die an dem Zugdraht 92 von dem Aktuator 32 geleistet wird, hängt zusammen mit der elektrischen Leistungsaufnahme des mechanischen Aktuators 32. Die Zugkräfte 100 und 102 können von der Steuereinheit 30 anhand der gemessenen Stromaufnahme und mittels Vergleichswerttabellen bestimmt werden.

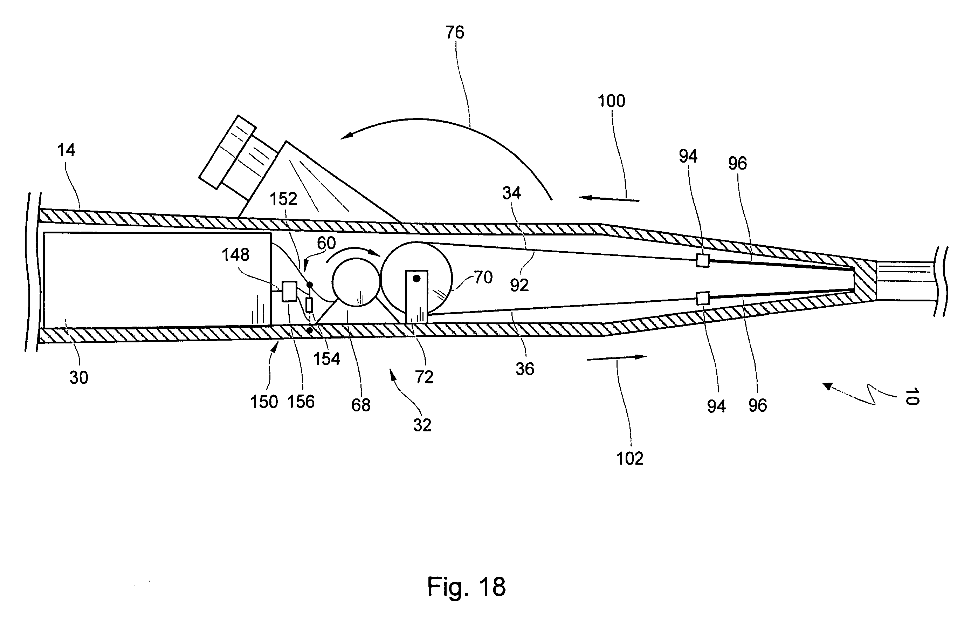

Figur 18 zeigt ein sechstes Ausführungsbeispiel des Endoskops 10. Es ist eine Vergrößerung der Schnittdarstellung des Endoskopgehäuses 14 dargestellt. Innerhalb des Endoskopgehäuses 14 befindet sich die Steuereinheit 30. Die Steuereinheit 30 ist über eine Leitung 148 mit der Messeinrichtung 60 verbunden. Die Messeinrichtung 60 besitzt einen Strommesssensor 150. Dieser weist einen Shuntwiderstand 154 und einen digitalen Spannungsmesssensor 156 auf. Der Spannungsmesssensor 156 misst eine elektrische Spannung an dem Shuntwiderstand 154. Der Shuntwiderstand 154 ist niederohmig ausgeführt, wobei ein Spannungsabfall an diesem Shuntwiderstand 154 proportional zu einem Versorgungsstrom zu dem Elektromotor 68 ist. Auf diese Weise bildet der Spannungsmesssensor 156 einen Strommesssensor. Die Spannung wird von den Spannungsmesssensor 156 erfasst und an das Steuergerät 30 weitergeleitet. Dabei weist der Spannungsmesssensor 156 einen A/D-Wandler auf, welcher hier nicht dargestellt ist. Für eine weitere Verarbeitung der erfassten Messwerte innerhalb der Steuereinheit 30 liegen damit die Messwerte in digitaler Form vor. Zur Bestimmung der Zugkraft 100 wird in der Steuereinheit 30 eine Drehmomentkonstante verwendet, welche sich individuell nach der konkreten Bauform des Elektromotors 68 bestimmt. Die Drehmomentkonstante steht in einem direkten Verhältnis zum Antriebsdrehmoment, welches von dem Elektromotor 68 geliefert wird. Innerhalb der Steuereinheit 30 lässt sich so auf sehr einfache Weise ein aufgewendetes Antriebsdrehmoment anhand des erfassten Versorgungsstroms in Verbindung mit der Drehmomentkonstante bestimmen. Durch Berücksichtigung des Radius der Seilrolle 70 kann anschließend die Zugkraft 100, 102 rechnerisch bestimmt werden. Somit lassen sich allein durch die Messung des Versorgungsstroms zum Elektromotor 68 die Zugkräfte 100, 102 bestimmen, wodurch ein sehr einfacher und wirtschaftlicher Aufbau des Endoskops 10 ermöglicht wird.

Zudem ist es in weiteren - hier nicht dargestellten - Ausführungsformen denkbar, dass zwischen der Seilrolle 70 und dem Elektromotor 68 ein Getriebe eingesetzt wird, in welchem ein Teil der vom Elektromotor 68 aufgewendeten Energie durch Reibung verlorengeht. Bei Verwendung eines solchen Getriebes ist bei der Berechnung der Wirkungsgrad des Getriebes zu berücksichtigen.

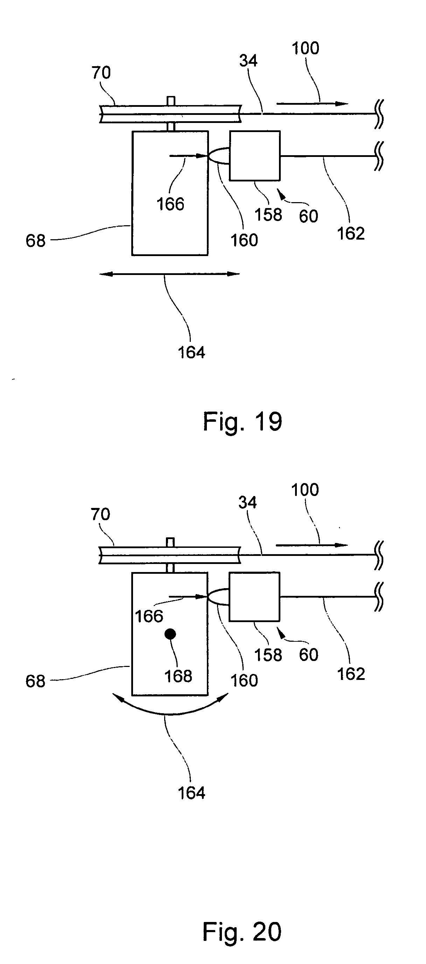

Figur 19 zeigt einen verlagerbaren Elektromotor 68, wobei der Übersicht halber auf die Darstellung des Endoskops 10 verzichtet wurde. Der Elektromotor 68 ist innerhalb des Endoskops angeordnet und ist mit der Seilrolle 70 verbunden, über die das erste Zugelement 34 bewegt werden kann. Das erste Zugelement 34 ist hier nur teilweise im Bereich des Elektromotors 68 dargestellt. An dem ersten Zugelement 34 liegt die Zugkraft 100 an, die hier in Richtung des distalen Endabschnitts 18 des Endoskops 10 zieht. Die Messeinrichtung 60 weist einen Kraftmesssensor 158 auf, der einen Taster 160 besitzt, welcher an dem Elektromotor 68 anliegt. Über eine Leitung 162 ist der Kraftmesssensor 158 mit der Steuereinheit 30 verbunden, welche hier nicht dargestellt ist. Der Elektromotor 68 ist in Richtung des Doppelpfeils 164 translatorisch verschiebbar gelagert. Somit kann der Elektromotor 68 in und gegen die Richtung der Zugkraft 100 verschoben werden. Durch die Zugkraft 100 wird der Elektromotor 68 in Richtung des distalen Endabschnitts verlagert und erzeugt dadurch eine Kraft entlang des Pfeils 166, welche auf den Taster 160 wirkt. Der Kraftmesssensor 158 ist gegenüber dem Elektromotor 68 ortsfest angeordnet, so dass die Kraft entlang des Pfeils 166 von dem Kraftmesssensor 158 gemessen und über die Leitung 162 der Steuereinheit 30 zugeführt werden kann, um dort beispielsweise für eine Regelung weiter verarbeitet zu werden.

Figur 20 zeigt eine weitere Ausführungsform des verlagerbaren Elektromotors 68. Der Elektromotor 68 weist eine Lagerung 168 auf, um die der Elektromotor 68 entlang des Doppelpfeils 164 verschwenkt werden kann. Die Verlagerung, in diesem Fall das Verschwenken, erfolgt aufgrund der Zugkraft 100. Durch diese rotatorische Verlagerung des Elektromotors 68 wird die Kraft 166 erzeugt, die auf den Taster 160 wirkt und somit ein Messen der Zugkraft 100 indirekt ermöglicht. Vorteilhaft hierbei ist, wenn die Detektionsweglänge des Kraftmesssensors 158 minimal gewählt wird, so dass die Schwenkbewegung des gesamten Elektromotors 68 unbedeutend gering ist, so dass das Zugelement 34 sicher innerhalb der Seilrolle 70 verbleibt und die Zugkraft 100 nicht wesentlich verändert wird.

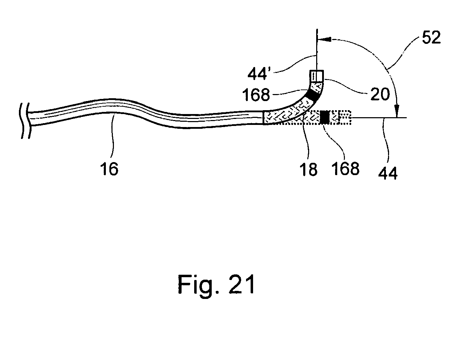

Figur 21 zeigt einen Bereich um den distalen Endabschnitt 18 des Endoskopschafts 16. In der ersten Momentanposition 44 ist der Endabschnitt 18 und das Endteil 20 gestrichelt dargestellt. In der zweiten Momentanposition 44' ist der Endabschnitt 18 sowie das Endteil 20 durchgehend dargestellt. Innerhalb des Endabschnitts 18 ist ein Krümmungssensor in Form eines Bewegungssensors 168 angeordnet, der die Krümmung des distalen Endabschnitts 18 direkt misst. Der Bewegungssensor 168 ist über hier nicht dargestellte Messleitungen mit der Steuereinheit 30 verbunden, um seine Messwerte an die Steuereinheit 30 zu übermitteln. Weiter ist der Bewegungssensor 168 hier als Beschleunigungssensor ausgeführt, welcher die Beschleunigung beim Verändern der Momentanposition 44, 44' aufnimmt. Dadurch ergibt sich die Möglichkeit innerhalb der Steuereinheit 30 die aktuelle Momentanposition 44' auf Basis der vorhergehenden Momentanposition 44 und der erfassten Beschleunigungen zu berechnen.

In weiteren Ausführungsformen sind unterschiedliche Arten an Krümmungssensoren denkbar, die auf unterschiedlichen Messprinzipien beruhen. Zum einen ist ein Geschwindigkeitssensor denkbar, welcher die Bewegungsgeschwindigkeit des Endabschnitts erfasst. Weiter sind Krümmungssensoren denkbar, die auf kapazitiver, induktiver oder ohmschen Veränderungen des Sensors durch die Krümmung selbst beruhen. Sie ermöglichen eine direkte Erfassung der Krümmung des Endabschnitts 18.

## Patentansprüche

1. Endoskop (10), das einen flexiblen Endoskopschaft (16) mit einem ablenkbaren distalen Endabschnitt (18) aufweist,
mit einem mechanischen Aktuator (32), der in einem proximalen Endbereich (28) des Endoskopschafts (16) angeordnet ist und der verschiedene Geschwindigkeitsstufen besitzt,
mit zumindest einem Zugelement (34, 36), das mit dem distalen Endabschnitt (18) und mit dem Aktuator (32) mechanisch gekuppelt ist, wobei der Aktuator (32) das Zugelement (34, 36) für eine Ablenkbewegung des distalen Endabschnitts (18) in eine Zugrichtung bewegt und
mit einer Steuereinheit (30), die dazu ausgebildet ist, den Aktuator (32) in seinen Geschwindigkeitsstufen zu steuern,
**dadurch gekennzeichnet, dass** die Steuereinheit (30) weiter dazu ausgebildet ist, den Aktuator (32) (zur Vermeidung eines Nachlaufwegs durch axiales Entspannen des Zugelements (34) am Ende der Ablenkbewegung dann in einer hohen Geschwindigkeitsstufe zu betreiben, wobei der Aktuator (32) das Zugelement (34, 36), für das Entspannen, gegen die Zugrichtung bewegt.

2. Endoskop (10) nach Anspruch 1, **dadurch gekennzeichnet, dass** die Steuereinheit (30) weiter dazu ausgebildet ist, den Aktuator (32) dann in der hohen Geschwindigkeitsstufe zu betreiben, wenn zu Beginn der Ablenkbewegung eine Anlauftotzeit durch axiales Spannen des Zugelements (34) besteht, wobei der Aktuator (32) das Zugelement (34, 36), für das Spannen, in die Zugrichtung bewegt.

3. Endoskop (10) nach Anspruch 2, **dadurch gekennzeichnet, dass** der Aktuator (32) zu Beginn der Ablenkbewegung ein Spanner (32') ist, indem der Aktuator (32) das Zugelement (34) um einen definierten Spannweg (80) verlagert.

4. Endoskop (10) nach einem der Ansprüche 1 bis 3, **dadurch gekennzeichnet, dass** der Aktuator (32) zum Ende der Ablenkbewegung ein Entspanner (32") ist, indem der Aktuator (32) das Zugelement (34) um einen definierten Entspannweg (84) verlagert.

5. Endoskop (10) nach einem der Ansprüche 1 bis 4, **gekennzeichnet durch** eine Ermittlungseinheit (56), die den Spannweg (80) oder den Entspannweg (84) in Abhängigkeit mindestens eines Parameters ermittelt.

6. Endoskop (10) nach Anspruch 5, **gekennzeichnet durch** eine Messeinrichtung (60), die mindestens einen der Parameter erfasst.

7. Endoskop (10) nach Anspruch 6, **dadurch gekennzeichnet, dass** die Messeinrichtung (60) mindestens einen Sensor (62) zur Messung der Position des Aktuators (32) aufweist.

8. Endoskop (10) nach einem der Ansprüche 6 oder 7, **dadurch gekennzeichnet, dass** die Messeinrichtung (60), mindestens einen Sensor (88, 90, 140, 142, 144, 146, 150, 156, 158) zur Messung einer Zugkraft (100, 102) in dem Zugelement (34, 36) aufweist.

9. Endoskop (10) nach Anspruch 8, **dadurch gekennzeichnet, dass** eine Anzahl der Sensoren (140, 142) als Piezoelement (140, 142) ausgebildet ist.

10. Endoskop (10) nach einem der Ansprüche 8 oder 9, **dadurch gekennzeichnet, dass** eine Anzahl der Sensoren (144, 146) als Dehnungsmessstreifen (144, 146) ausgebildet ist.

11. Endoskop (10) nach einem der Ansprüche 8 bis 10, **dadurch gekennzeichnet, dass** mindestens einer der Sensoren (150) als Strommesssensor (150, 156) ausgebildet ist, der einen elektrischen Strom zu dem Aktuator (32) erfasst.

12. Endoskop (10) nach Anspruch 11, **dadurch gekennzeichnet, dass** der Strommesssensor (150) einen Spannungsmesssensor (156) und einen Shuntwiderstand (154) aufweist, wobei der Spannungsmesssensor (156) eine elektrische Spannung am Shuntwiderstand (154) erfasst.

13. Endoskop (10) nach einem der Ansprüche 8 bis 12, **dadurch gekennzeichnet, dass** eine Anzahl der Sensoren (88, 90) als Zugschalter (88, 90) ausgebildet ist.

14. Endoskop (10) nach einem der Ansprüche 8 bis 13, **dadurch gekennzeichnet, dass** mindestens einer der Sensoren (88, 90) einen ersten ohmschen Widerstand (114) besitzt, dessen Widerstandswert dem Sensor (88, 90) eindeutig zugeordnet ist.

15. Endoskop (10) nach Anspruch 14, **dadurch gekennzeichnet, dass** der Sensor (88, 90) als Zugschalter ausgebildet ist und den ersten ohmschen Widerstand (114) für eine erste Schaltposition besitzt.

16. Endoskop (10) nach Anspruch 15, **dadurch gekennzeichnet, dass** der Zugschalter (88) einen zweiten ohmschen Widerstand (116) für eine zweite Schaltposition besitzt, wobei sich der Widerstandswert des zweiten Widerstandes (116) von dem Widerstandswert des ersten Widerstandes (114) unterscheidet.

17. Endoskop (10) nach einem der Ansprüche 8 bis 16, **dadurch gekennzeichnet, dass** das Zugelement (34, 36) eine elektrische Leitung für den Sensor (88, 90, 140, 142, 144, 146, 150) bildet.

18. Endoskop (10) nach einem der Ansprüche 8 bis 17, **dadurch gekennzeichnet, dass** das Zugelement (34, 36) eine Signalleitung für den Sensor (88, 90, 140, 142, 144, 146, 150) bildet.

19. Endoskop (10) nach einem der Ansprüche 8 bis 18, **dadurch gekennzeichnet, dass** der Aktuator (68) verlagerbar zu der Messeinrichtung (60) angeordnet ist und zur Messung der Zugkraft (100) in dem Zugelement mit dem Sensor (158) zusammenwirkt.

20. Endoskop (10) nach einem der Ansprüche 6 bis 19, **dadurch gekennzeichnet, dass** die Messeinrichtung (60) mindestens einen Krümmungssensor (168) zur Messung einer Krümmung des distalen Endabschnitts (18) aufweist.

21. Endoskop (10) nach Anspruch 20, **dadurch gekennzeichnet, dass** der Krümmungssensor (168) ein Biegesensor ist.

22. Endoskop (10) nach einem der Ansprüche 20 oder 21, **dadurch gekennzeichnet, dass** der Krümmungssensor (168) ein Bewegungssensor (168) ist.

## Claims

1. Endoscope (10) comprising a flexible endoscope shaft (16) with a deflectable distal end section (18),
comprising a mechanical actuator (32) arranged in a proximal end section (28) of the endoscope shaft (16) and having different levels of speed,
comprising at least one pull element (34, 36) which is mechanically coupled with the distal end section (18) and with the actuator (32), wherein the actuator (32) moves the pull element (34, 36) for a deflecting movement of the distal end section (18) into a pull direction, and
comprising a control unit (30), which is configured to control the actuator (32) regarding its levels of speed,
**characterized in that** the control unit (30) is further configured to drive the actuator for avoiding a lagging path caused by axial relaxation of the pull element (34) at the end of the deflecting movement then at a high level of speed, wherein the actuator (32) moves the pull element (34, 36) for the relaxation against the pull direction.

2. Endoscope (10) according to claim 1, **characterized in that** the control unit (30) is further configured to drive the actuator (32) then at the high level of speed, when at the beginning of the deflecting movement a start up reaction time caused by an axial tensioning of the pull element (34) is present, wherein the actuator (32) moves the pull element (34, 36) into the pull direction for the tensioning.

3. Endoscope (10) according to claim 2, **characterized in that** the actuator (32) is a tensioning element (32') at the beginning of the deflecting movement due to the actuator displacing the pull element (34) by a defined tensioning path (80).

4. Endoscope (10) according to any of claims 1 to 3, **characterized in that** the actuator is a relaxing element (32") at the end of the deflecting movement due to the actuator (32) displacing the pull element (34) by a defined relaxation path (84).

5. Endoscope (10) according to any of claims 1 to 4, **characterized by** a detection unit (56) which detects the tensioning path (80) or the relaxing path (84) depending at least on one parameter.

6. Endoscope (10) according to claim 5, **characterized by** a measuring unit (60) which captures at least one of the parameters.

7. Endoscope (10) according to claim 6, **characterized in that** the measuring unit (60) comprises at least one sensor (62) for measuring the position of the actuator (32).

8. Endoscope (10) according to one of claims 6 or 7, **characterized in that** the measuring unit (60) comprises at least one sensor (88, 90, 140, 142, 144, 146, 150, 156, 158) for measuring a pull force (100, 102) in the pull element (34, 36).

9. Endoscope (10) according to claim 8, **characterized in that** a number of the sensors (140, 142) is embodied as a piezo element (140, 142).

10. Endoscope (10) according to any of claims 8 or 9, **characterized in that** a number of the sensors (144, 146) is embodied as a strain gauge (144, 146)

11. Endoscope (10) according to any of claims 8 to 10, **characterized in that** at least one of the sensors (150) is embodied as a current measuring sensor (150, 156) which captures an electrical current to the actuator (32).

12. Endoscope (10) according to claim 11, **characterized in that** the current measuring sensor (150) comprises a voltage measuring sensor (156) and an electric shunt (154), wherein the voltage measuring sensor (156) captures an electrical voltage at the electric shunt (154).

13. Endoscope (10) according to any of claims 8 to 12, **characterized in that** a number of the sensors (88, 90) is embodied as a pull switch (80, 90).

14. Endoscope (10) according to any of claims 8 to 13, **characterized in that** at least one of the sensors (88, 90) comprises a first ohmic resistor (114), its resistor value unambiguously assigned to the sensor (88, 90).

15. Endoscope (10) according to claim 14, **characterized in that** the sensor (88, 90) is embodied as a pull switch and has the first ohmic resistor (114) for a first switching position.

16. Endoscope (10) according to claim 15, **characterized in that** the pull switch (88) has a second ohmic resistor (116) for a second switching position, wherein the resistor value of the second resistor (116) is different from the resistor value of the first resistor (114).

17. Endoscope (10) according to any of claims 8 to 16, **characterized in that** the pull element (34, 36) forms an electrical conductor for the sensor (88, 90, 140, 142, 144, 146, 150).

18. Endoscope (10) according to any claims 8 to 17, **characterized in that** the pull element (34, 36) forms a signal line for the sensor (88, 90, 140, 142, 144, 146, 150).

19. Endoscope (10) according to any of claims 8 to 18, **characterized in that** the actuator (68) is arranged in a manner displaceable to the measuring unit (60) and cooperates with the sensor (158) for measuring the pull force (100) in the pull element.

20. Endoscope (10) according to any of claims 6 to 19, **characterized in that** the measuring unit (60) comprises at least one curvature sensor (168) for measuring a curvature of the distal end section (18).

21. Endoscope (10) according to claim 20, **characterized in that** the curvature sensor (168) is a bending sensor.

22. Endoscope (10) according to any of claims 20 or 21, **characterized in that** the curvature sensor (168) is a movement sensor (168).

## Revendications

1. Endoscope (10) qui présente
une tige flexible (16) d'endoscope dotée d'une partie d'extrémité distale (18) apte à être déviée,
un actionneur mécanique (32) disposé dans une partie d'extrémité proximale (28) de la tige d'endoscope (16) et qui possède différents étages de vitesse,
au moins un élément de traction (34, 36) accouplé mécaniquement à la partie d'extrémité distale (18) et à l'actionneur (32),
l'actionneur (32) déplaçant l'élément de traction (34, 36) dans une direction de traction en vue d'un déplacement de déviation de la partie d'extrémité distale (18) et
une unité de commande (30) configurée pour commander l'actionneur (32) dans ses étages e vitesse, **caractérisé en ce que**
en vue d'éviter un déplacement secondaire, l'unité de commande (30) est en outre configurée pour actionner l'actionneur (32) dans un étage de vitesse élevée par détente axiale de l'élément de traction (34) à la fin du déplacement de déviation, l'actionneur (32) déplaçant pour la détente l'élément de traction (34, 36) en opposition à la direction de traction.

2. Endoscope (10) selon la revendication 1, **caractérisé en ce que** l'unité de commande (30) est en outre configurée pour actionner l'actionneur (32) à l'étage de vitesse élevée si au début du déplacement de déviation, un temps mort de démarrage est présent suite à la tension axiale de l'élément de traction (34), l'actionneur (32) déplaçant l'élément de traction (34, 36) dans la direction de traction en vue de la tension.

3. Endoscope (10) selon la revendication 2, **caractérisé en ce que** l'actionneur (32) est un dispositif de serrage (32') au début du déplacement de déviation par le fait que l'actionneur (32) déplace l'élément de traction (34) sur un parcours de serrage (80) défini.

4. Endoscope (10) selon l'une des revendications 1 à 3, **caractérisé en ce qu'**à la fin du déplacement de déviation, l'actionneur (32) est un dispositif de détente (32") par le fait que l'actionneur (32) déplace l'élément de traction (34) sur un parcours de détente (84) défini.

5. Endoscope (10) selon l'une des revendications 1 à 4, **caractérisé par** une unité de détermination (56) qui détermine le parcours de serrage (80) ou le parcours de détente (84) en fonction d'au moins un paramètre.

6. Endoscope (10) selon la revendication 5, **caractérisé par** un dispositif de mesure (60) qui saisit au moins un des paramètres.

7. Endoscope (10) selon la revendication 6, **caractérisé en ce que** le dispositif de mesure (60) présente au moins un détecteur (62) qui mesure la position de l'actionneur (32).

8. Endoscope (10) selon l'une des revendications 6 ou 7, **caractérisé en ce que** le dispositif de mesure (60) présente au moins un détecteur (88, 90, 140, 142, 144, 146, 150, 156, 158) destiné à mesurer une force de traction (100, 102) dans l'élément de traction (34, 36).

9. Endoscope (10) selon la revendication 8, **caractérisé en ce que** plusieurs des détecteurs (140, 142) sont configurés comme piézoéléments (140, 142).

10. Endoscope (10) selon l'une des revendications 8 ou 9, **caractérisé en ce que** plusieurs des détecteurs (144, 146) sont configurés comme rubans (144, 146) de mesure d'allongement.

11. Endoscope (10) selon l'une des revendications 8 à 10, **caractérisé en ce qu'**au moins l'un des détecteurs (150) est configuré comme détecteur (150, 156) de mesure de courant qui saisit un courant électrique envoyé à l'actionneur (32).

12. Endoscope (10) selon la revendication 11, **caractérisé en ce que** le détecteur (150) de mesure de courant présente un détecteur (156) de mesure de tension et une résistance de shunt (154), le détecteur (156) de mesure de tension saisissant la tension électrique aux bornes de la résistance de shunt (154).

13. Endoscope (10) selon l'une des revendications 8 à 12, **caractérisé en ce que** plusieurs des détecteurs (88, 90) sont configurés comme commutateurs de traction (88, 90).

14. Endoscope (10) selon l'une des revendications 8 à 13, **caractérisé en ce qu'**au moins l'un des détecteurs (88, 90) possède une première résistance ohmique (114) dont la valeur de la résistance est associée de manière univoque au détecteur (88, 90).

15. Endoscope (10) selon la revendication 14, **caractérisé en ce que** le détecteur (88, 90) est configuré comme commutateur de traction et possède la première résistance ohmique (114) pour une première position de commutation.

16. Endoscope (10) selon la revendication 15, **caractérisé en ce que** le commutateur de traction (88) possède une deuxième résistance ohmique (116) pour une deuxième position de commutation, la valeur de la deuxième résistance (116) étant différente de la valeur de la première résistance (114).

17. Endoscope (10) selon l'une des revendications 8 à 16, **caractérisé en ce que** l'élément de traction (34, 36) forme un conducteur électrique pour le détecteur (88, 90, 140, 142, 144, 146, 150).

18. Endoscope (10) selon l'une des revendications 8 à 17, **caractérisé en ce que** l'élément de traction (34, 36) forme un conducteur de signalisation pour le détecteur (88, 90, 140, 142, 144, 146, 150).

19. Endoscope (10) selon l'une des revendications 8 à 18, **caractérisé en ce que** l'actionneur (68) est disposé de manière à pouvoir être déplacé par rapport au dispositif de mesure (60) et coopère avec le détecteur (158) pour mesurer la force de traction (100) appliquée dans l'élément de traction.

20. Endoscope (10) selon l'une des revendications 6 à 19, **caractérisé en ce que** le dispositif de mesure (60) présente au moins un détecteur de courbure (168) qui mesure la courbure de la partie d'extrémité distale (18).

21. Endoscope (10) selon la revendication 20, **caractérisé en ce que** le détecteur de courbure (168) est un détecteur de flexion.

22. Endoscope (10) selon l'une des revendications 20 ou 21, **caractérisé en ce que** le détecteur de courbure (168) est un détecteur de déplacement (168).
